# EUROPEAN PATENT APPLICATION

(11) **EP 1 553 451 A1**
(43) Date of publication of application: **13.07.2005**
(21) Application number: 03808872.0
(22) Date of filing: 01.09.2003
(51) Int. Cl.: G03F 7/039, C07C 39/17, C07C 69/736, C07D 309/04

(54) **PHOTORESIST BASE MATERIAL, METHOD FOR PURIFICATION THEREOF, AND PHOTORESIST COMPOSITIONS**

(30) Priority: 15.10.2002 JP 2002300144; 17.04.2003 JP 2003112458
(71) Applicant: IDEMITSU KOSAN CO., LTD., Tokyo 100-8321 (JP)
(72) Inventor: UEDA, Mitsuru, Meguro-ku, Tokyo 152-8550 (JP); ISHII, Hirotoshi, Ichihara-shi, Chiba 299-0107 (JP)
(74) Representative: Gille, Struck, Neidlein, Prop, Roos
(86) International application number: PCT/JP2003/011137
(87) International publication number: WO 2004/036315

(57) **Abstract**

A photoresist base material comprising an extreme ultra-violet reactive organic compound of the following formula (1), 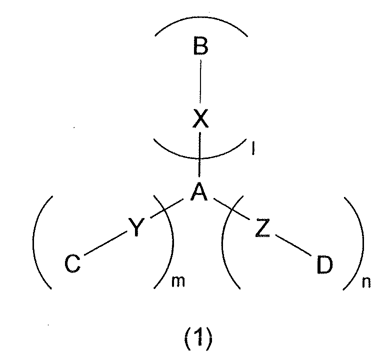 wherein A is a central structure that is an aliphatic group having 1 to 50 carbon atoms, an aromatic group having 6 to 50 carbon atoms, an organic group containing these together or an organic group having a cyclic structure formed by repetition of these, each of B to D is an extreme ultra-violet reactive group, a group having reactivity to the action of a chromophore active to extreme ultra-violet, or a C₁ to C₅₀ aliphatic group, C₆ to C₅₀ aromatic group, an organic group containing these together or a substituent having a branched structure, containing such a reactive group, X to Z are single bonds or ether bonds, l to n are integers of 0 to 5 satisfying l + m + n ≥ 1, and A to D may contain a substituent having a heteroatom. The photoresist base material and a composition thereof enable ultrafine processing based on extreme ultra-violet.

## Description

### Technical Field

The present invention relates to a photoresist base material for use in electric-electronic fields of semiconductors, etc., and optical fields, particularly, a photoresist base material for ultrafine processing, a method for purification thereof, and a photoresist composition.

### Technical Background

Lithography based on extreme ultra-violet (EUV), vacuum ultra-violet, electron beam, ion beam, etc., particularly, lithography based on extreme ultra-violet or electron beam is useful as a highly productive ultrafine processing technique in the fabrication of semiconductors, and the like. It is demanded to develop a high-sensitivity high-resolution photoresist that copes with 100 nm or finer, particularly 50 nm or finer processing by means of extreme ultra-violet or electron beam. Concerning a photoresist for use in the above lithography, it is essential to improve the photoresist in sensitivity from the viewpoint of the productivity, resolution, etc., of desired fine patterns.

As a photoresist for use in the ultrafine processing based on extreme ultra-violet, a chemical amplification type polyhydroxystyrene-based photoresist is exemplified that has been used for ultrafine processing with a known KrF laser. It is known that the above resist permits fine processing up to approximately 50 nm (for example, Surveillance studies on next-generation EUVL (Extreme Ultra-Violet Lithography) technique, Investigative Report of fiscal year of 2001, Project sponsored by New Energy and Industrial Technology Development Organization). However, when 50 nm or finer patterns that is the greatest advantage of ultrafine processing based on extreme ultra-violet are made, this resist causes problems of a low contrast, a large line-edge roughness, a low resist sensitivity, a large amount of a resist outgas, etc., so that performances inherent to the extreme ultra-violet have been fully brought out. Under the circumstances, it has been demanded to develop a higher-performance photoresist for use with extreme ultra-violet.

For coping with this demand, there is proposed a method using a chemical amplification type photoresist having a high concentration of an optically-acid-generating compound (e.g., JP-A-2002-055457). In this method, however, Examples do not show any specific results of sensitivity, line-edge roughness, a created line width, etc., in the case of using extreme ultra-violet with regard to a photoresist containing a base material that is a hydroxystyrene/styrene/t-butyl acrylate terpolymer, an optically-acid-generating agent that is di(t-butylphenyl)iodonium o-trifluoromethyl sulfonate contained in an amount of at least about 5 % based on a total solid content, tetrabutylammonium hydroxide lactate and ethyl lactate. Based on these results, from the viewpoint of line-edge roughness, it has been therefore considered that processing as fine as 100 nm as is shown in the case of using an electron beam is the limit. It is assumed to be caused by an excess reaction of the base material due to an excess addition of the optically-acid-generating agent, that is, excess diffusion of an acid into a non-exposed portion.

While fine processing based on electron beam is inferior to that using extreme ultra-violet in productivity, it is suitable for fabricating special semiconductors of which the lifetime production quantity is small, since it does not require a mask. As a photoresist for use with electron beam, both positive type and negative type photoresists of many kinds are proposed, and it is said that an isolated line having a width of 8 nm can be created when an electron beam irradiation apparatus having a sufficiently small focusing radius is used. In this case, however, the molecular form of a polymer compound contained as a base material is reflected in the line-edge roughness, a line width is not sufficiently fine.

With developments in far finer processing of semiconductors, severer values are required concerning the line-edge roughness. In conventional photoresists containing a polymer compound as a base material, however, the molecular form thereof is reflected in the line-edge roughness. In the region of 50 nm or finer ultrafine processing, therefore, it has been theoretically difficult to carry out the processing while limiting the line-edge roughness, for example, to 3 nm or less when a polymer compound having a molecular size of approximately several tens nm is used.

The present invention has been made in view of the above circumstances. It is an object of the present invention to provide a photoresist base material that enables ultrafine processing based on extreme ultra-violet, or the like with high sensitivity, a high contrast and a low line-edge roughness, a method for purification thereof and a photoresist composition.

For achieving the above object, the present inventors have made diligent studies and as a result have found that the a decrease in the sensitivity of a photoresist is caused by basic impurities such as ammonia, alkali metal ion, alkaline earth metal ion, etc., that are remaining since they are used in synthesis or that are incorporated from a human body or surroundings.

In the case where a photoresist absorbs extreme ultra-violet in a high ratio when extreme ultra-violet passes therethrough and the intensity of a light source is low, an optically-acid-generating agent may be added at a high concentration. When even a trace amount of a basic impurity is included therein, it neutralizes proton generated from the acid-generating agent, so that no desired reaction can proceed.

The present inventors have found that when a photoresist base material is purified so that the basic impurities are removed to a certain value or less, the sensitivity is remarkably increased, and the present invention has been accordingly completed.

### Disclosure of the Invention

According to the present invention, there are provided the following photoresist base material, and the like.
[1] A photoresist base material comprising an extreme ultra-violet reactive organic compound represented by the following general formula (1), 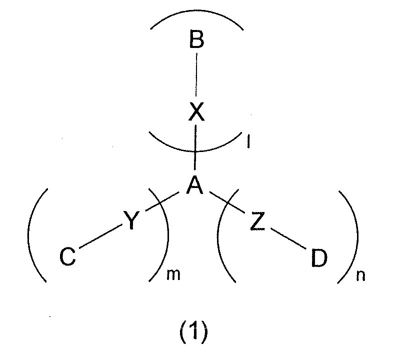 wherein A is a central structure that is an aliphatic group having 1 to 50 carbon atoms, an aromatic group having 6 to 50 carbon atoms, an organic group containing said aliphatic group and said aromatic group together or an organic group having a cyclic structure formed by repetition of these groups, each of B, C and D is independently an extreme ultra-violet reactive group, a group having reactivity to the action of chromophore sensitive to extreme ultra-violet, or a C₁ to C₅₀ aliphatic group, C₆ to C₅₀ aromatic group, an organic group containing said aliphatic group and said aromatic group together or a substituent having a branched structure, containing such a reactive group, each of X, Y and Z is independently a single bond or an ether bond, each of l, m and n is independently an integer of 0 to 5 satisfying l + m + n ≥ 1, and A, B, C and D may contain a substituent having a heteroatom.
[2] The photoresist base material as recited in [1], wherein said organic compound reactive to extreme ultra-violet is in an amorphous state at room temperature and has a molecule whose average diameter is 2 nm or less.
[3] The photoresist base material as recited in [1] or [2], wherein A is an organic group represented by 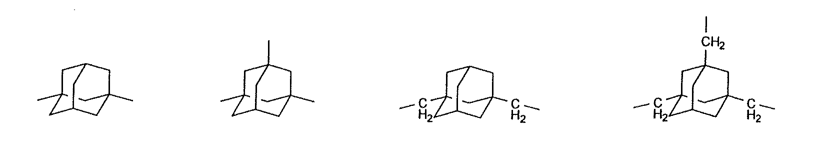 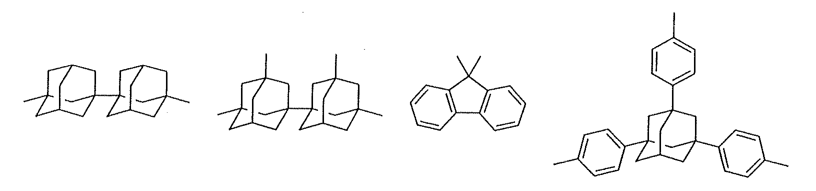 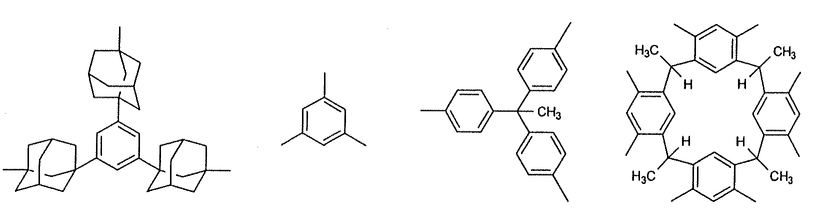 each of B, C and D is an extreme ultra-violet reactive group, a group having reactivity to the action of chromophore active to extreme ultra-violet, or an organic group represented by 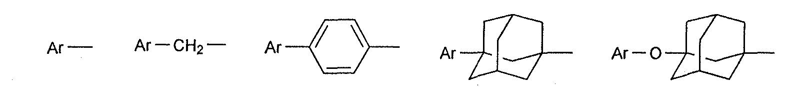 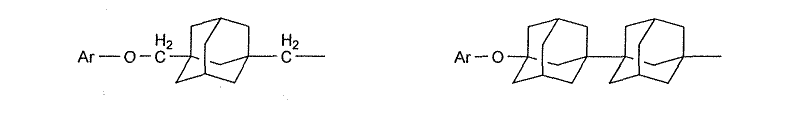 wherein Ar is a phenyl or naphthyl group substituted with RO- and/or ROCO- in which R, RO- and ROCO are extreme ultra-violet reactive groups or groups having reactivity to the action of a chromophore active to extreme ultra-violet, and
   X, Y and Z are ether bonds.
[4] The photoresist base material as recited in [3], wherein A is an organic group represented by 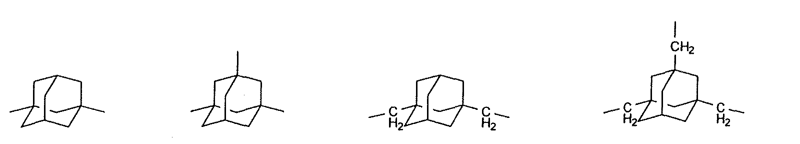 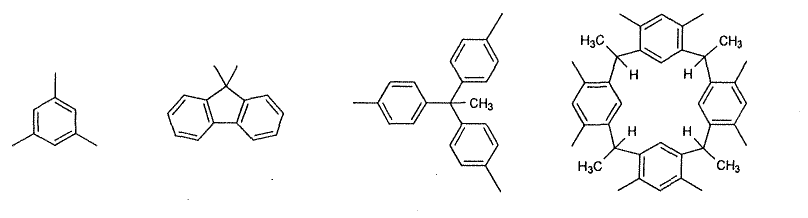 each of B, C and D is a hydrogen atom, tert-butyl, tert-butyloxycarbonylmethyl, tert-butyloxycarbonyl, 1-tetrahydropyranyl, 1-tetrahydrofuranyl, 1-ethoxyethyl, 1-phenoxyethyl, an organic group represented by 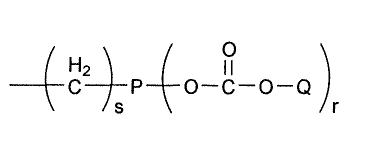 in which P is an aromatic group having a valence of (r + 1) and having 6 to 20 carbon atoms, Q is an organic group having 4 to 30 carbon atoms, r is an integer of 1 to 10 and s is an integer of 0 to 10, or an organic group represented by 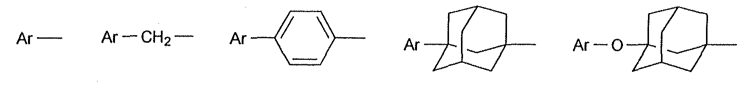 in which Ar is a phenyl or naphthyl group substituted with RO- and/or ROCO- in which R is hydrogen, tert-butyl, tert-butyloxycarbonylmethyl, tert-butyloxycarbonyl, 1-tetrahydropyranyl, 1-tetrahydrofuranyl, 1-ethoxyethyl, 1-phenoxyethyl or an organic group represented by 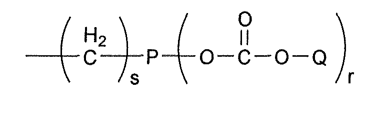 in which P is an aromatic group having a valence of (r + 1) and having 6 to 20 carbon atoms, Q is an organic group having 4 to 30 carbon atoms, r is an integer of 1 to 10 and s is an integer of 0 to 10,
   and X, Y and Z are ether bonds.
[5] The photoresist base material as recited in [4], wherein A is an organic group represented by 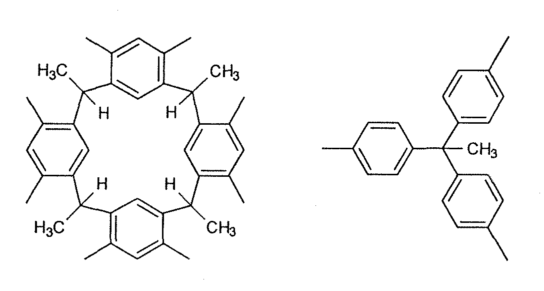 each of B, C and D is a hydrogen atom, tert-butyl, tert-butyloxycarbonylmethyl, tert-butyloxycarbonyl, 1-tetrahydropyranyl, 1-tetrahydrofuranyl, 1-ethoxyethyl, 1-phenoxyethyl or an organic group represented by 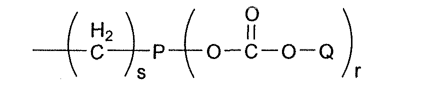 in which P is an aromatic group having a valence of (r + 1) and having 6 to 20 carbon atoms, Q is an organic group having 4 to 30 carbon atoms, r is an integer of 1 to 10 and s is an integer of 0 to 10,
   and X, Y and Z are ether bonds.
[6] A photoresist base material comprising a radiation-sensitive organic compound represented by the following general formula (1), 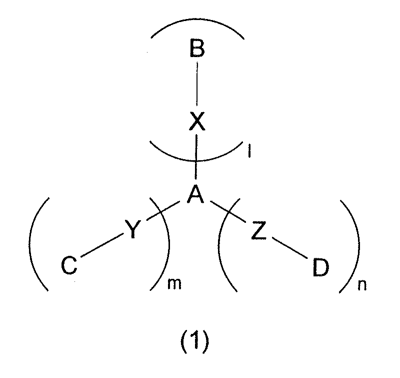 wherein A is an organic group represented by 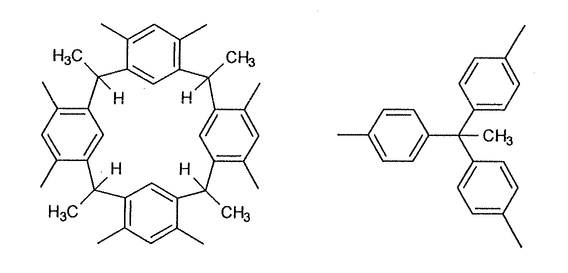 each of B, C and D is independently an organic group represented by 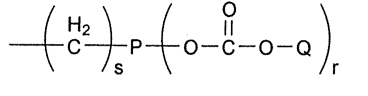 in which P is an aromatic group having a valence of (r + 1) and having 6 to 20 carbon atoms, Q is an organic group having 4 to 30 carbon atoms, r is an integer of 1 to 10 and s is an integer of 0 to 10, and
   l + m + n = 3 or 8.
[7] The photoresist base material as recited in any one of the above [4] to [6], wherein the organic group represented by 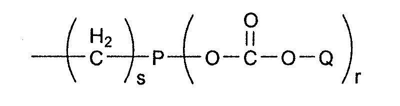 is 4-(tert-butoxycarbonyloxy)benzyl or 3,5-di(tert-butoxycarbonyloxy)benzyl.
[8] The photoresist base material as recited in [6] or [7], wherein the radiation is extreme ultra-violet or electron beam.
[9] The photoresist base material as recited in any one of [1] to [8], wherein at least one of B, C and D is a hydrogen atom and X, Y and Z are ether bonds.
[10] The photoresist base material as recited in any one of [1] to [9], which has a basic impurity content of 10 ppm or less.
[11] A photoresist composition containing a solid content containing the photoresist base material recited in any one of [1] to [10], and a solvent.
[12] The photoresist composition as recited in [11], which further contains an optically-acid-generating agent.
[13] A method for purification of a photoresist base material, which comprises washing the photoresist base material recited in any one of [1] to [9] with an acidic aqueous solution and treating the material with an ion-exchange resin.
[14] The method for purification of a photoresist base material as recited in [13], wherein said acidic aqueous solution is an acetic acid aqueous solution.
[15] A method for improvement of the photoresist base material recited in any one of [1] to [9] in radiation sensitivity, which comprises decreasing the content of basic impurities to 10 ppm or less.
[16] A method for fine processing by lithography, which uses the photoresist composition recited in [11] or [12] .
[17] A semiconductor device fabricated using the photoresist composition recited in [11] or [12].
[18] An organic compound represented by the following general formula (1), 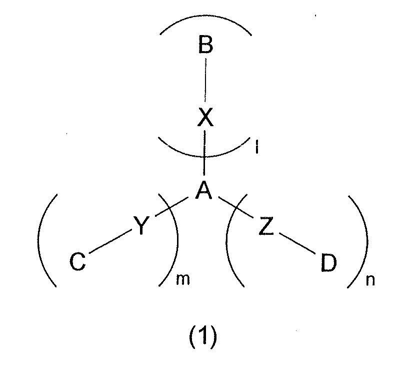 wherein A is an organic group represented by 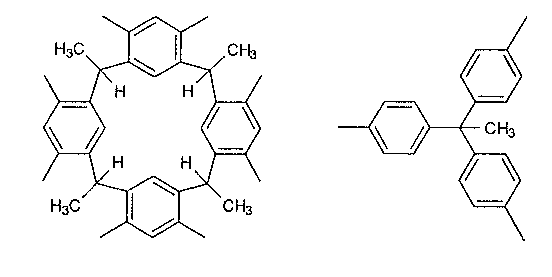 each of B, C and D is independently an organic group represented by 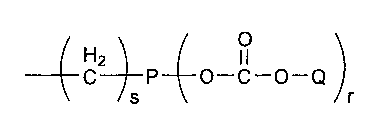 in which P is an aromatic group having a valence of (r + 1) and having 6 to 20 carbon atoms, Q is an organic group having 4 to 30 carbon atoms, r is an integer of 1 to 10 and s is an integer of 0 to 10, and
   l + m + n = 3 or 8.
[19] The organic compound as recited in [18], which has a basic impurity content of 10 ppm or less.
[20] A method for purification of an organic compound, which comprises washing the organic compound recited in [18] with an acidic aqueous solution and treating the compound with an ion-exchange resin.

### Best Modes for Carrying out the Invention

The photoresist base material of the present invention will be explained hereinafter.

The photoresist base material of the present invention comprises an organic compound represented by the following general formula (1). This compound is radiation-sensitive.

The above radiation refers to ultraviolet rays having a wavelength of 10 to 300 nm, specifically, extreme ultra-violet and vacuum ultraviolet, or an electron beam, an ion beam, or the like.

The compound for use in the present invention is preferably reactive with extreme ultra-violet and/or electron beams, more preferably reactive with extreme ultra-violet. In addition, this compound is also reactive with other general radiations (such as infrared light, visible light ultraviolet light, ultraviolet light (g ray, i ray, etc.), X ray, etc.). 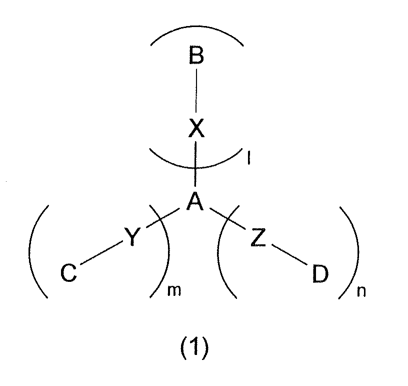 wherein A is a central structure that is an aliphatic group having 1 to 50 carbon atoms, an aromatic group having 6 to 50 carbon atoms, an organic group containing these aliphatic and aromatic groups together or an organic group having a cyclic structure formed by repetition of these groups, each of B, C and D is independently a radiation-sensitive group, a group having reactivity to the action of chromophore active to radiation, or a C₁ to C₅₀ aliphatic group, C₆ to C₅₀ aromatic group, an organic group containing these aliphatic and aromatic groups together or a substituent having a branched structure, containing such a reactive group, each of X, Y and Z is independently a single bond or an ether bond, each of l, m and n is independently an integer of 0 to 5 satisfying l + m + n ≥ 1, and A, B, C and D may contain a substituent having a heteroatom.

In the central structure A, examples of the C₁ to C₅₀ aliphatic group include linear aliphatic groups such as methyl, methylene, ethyl, dodecyl, etc., branched aliphatic groups such as isopropyl, isobutyl, tert-butyl, etc., and cyclic aliphatic groups such as cyclohexyl, norbornenyl, adamantyl, diadamantyl, biadamantyl, and the like. Examples of the C₆ to C₅₀ aromatic group include phenyl, phenylene, naphthyl, naphthylene, fluorene, alkyl aromatic group, and the like. Examples of the organic group containing these aliphatic and aromatic groups together include a group containing adamantyl and a methylene group together and a group containing adamantyl and phenylene group together. Examples of the organic group having a cyclic structure formed by repetition of these groups include calixarenes such as calix-[4]-resorcinarene, and the like. Examples of the substituent having a heteroatom include acetal groups such as 1-tetrahydropyranyl, 1-tetrahydrofuranyl, methoxymethyl and ethoxyethyl, carbonyl, hydroxyl, carboxyl, a t-butyl ester group, an alkoxy group, a cyano group, an amino group, an amido group, an imido group, pyridyl, and the like. The central structure A may be a single organic group above, or may be a group formed by a combination of a plurality of the same or different organic groups above.

Specific examples of the central structure A include organic groups shown below.

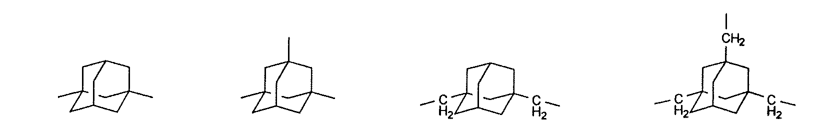

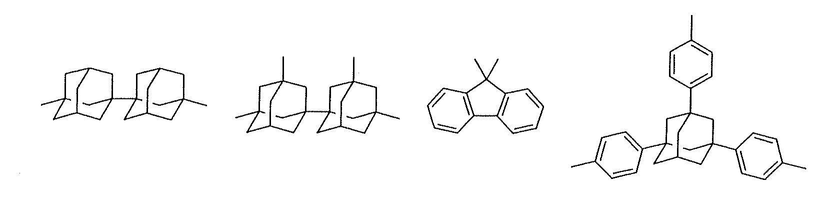

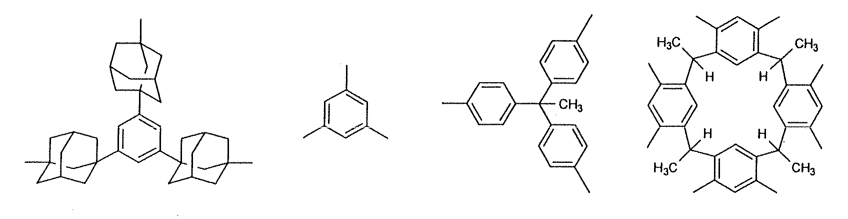

Each of B to D represents a substituent that exhibits reactivity upon irradiation with radiation (radiation-sensitive group), a group having reactivity to a chromophore active to radiation or a substituent containing these. In these substituents B to D, the C₁ to C₅₀ aliphatic group, the C₆ to C₆₀ aromatic group, the organic group containing these aliphatic and aromatic groups together and the substituent having a heteroatom include those explained with regard to the central structure A. Further, examples of the substituent having a branched structure include groups typified by dendron. The positions of the substituents B to D on the central structure A are not limited.

Of B, C and D, preferably, at least one of them is a hydrogen atom.

Specific examples of the substituents B to D include the following organic groups, radiation-sensitive groups to be described later and groups R, RO- and ROCO-which have reactivity to the action of chromophore active to radiation.

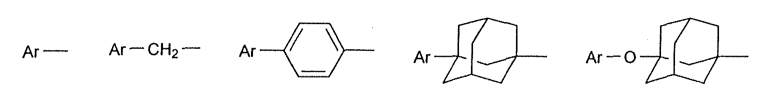

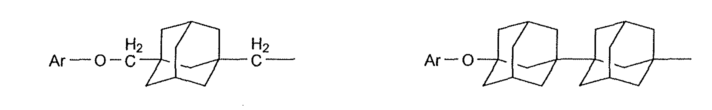

Ar is phenyl or naphthyl substituted with RO-and/or ROCO in which each of R, RO- and ROCO- is a radiation-sensitive group or a group having reactivity to the action of a chromophore active to radiation.

Specifically, the compound for use in the present invention includes compounds of the following formulae (2) to (17), position isomers thereof, and the like.

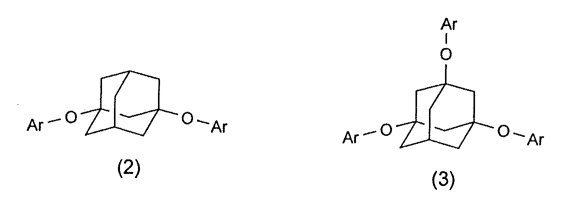

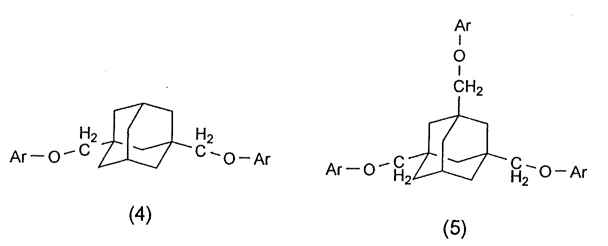

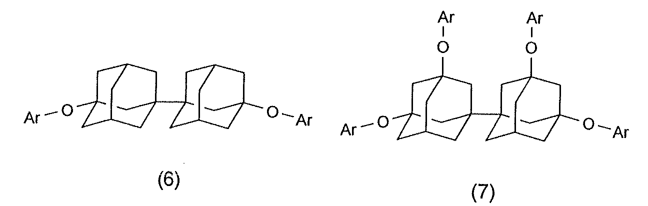

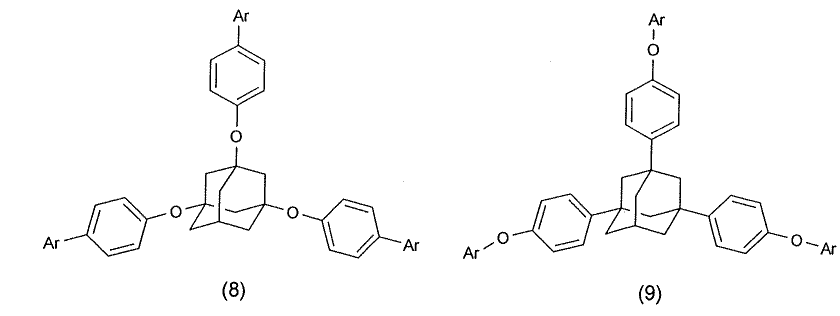

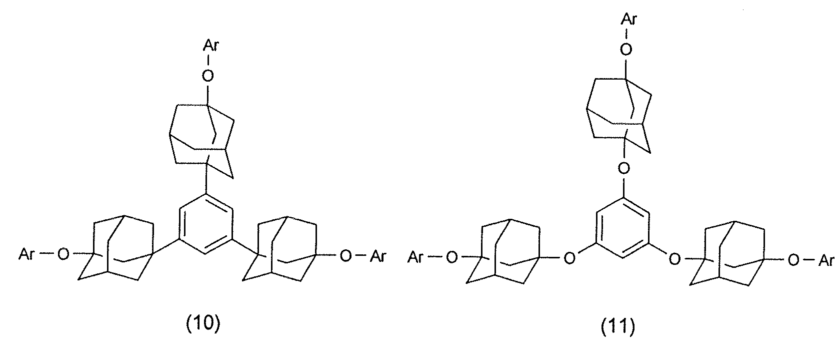

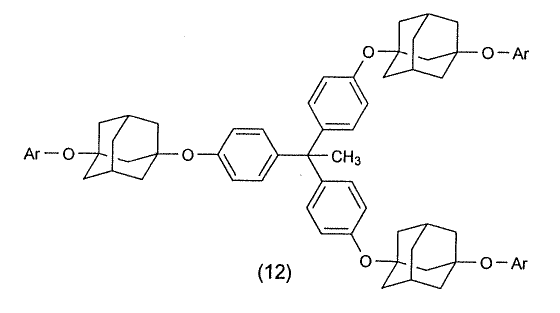

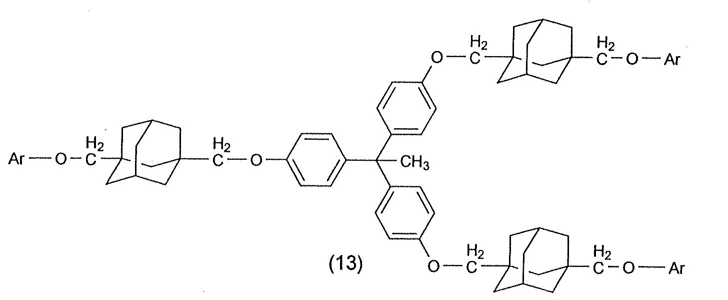

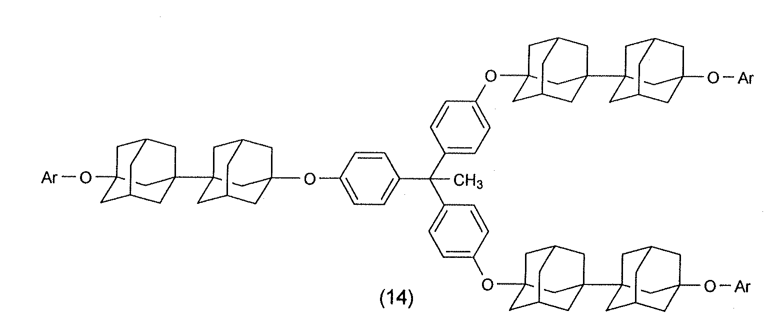

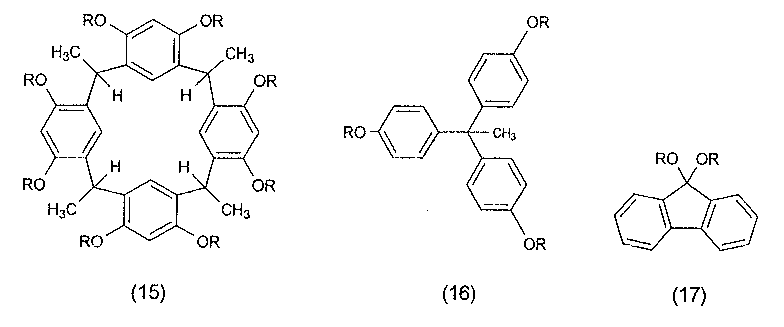

In the compounds of the above formulae (2) to (14), any substituent Ar can be suitably used so long as the substituent Ar contains a radiation-sensitive group or a group R, RO- or ROCO- having reactivity to the action of a chromophore active to radiation (to be described later). Specifically, Ar includes the following substituents and position isomers thereof. Substituents Ar may be used singly or may be used in combination of two or more members thereof so long as an advantage of the present invention is not impaired.

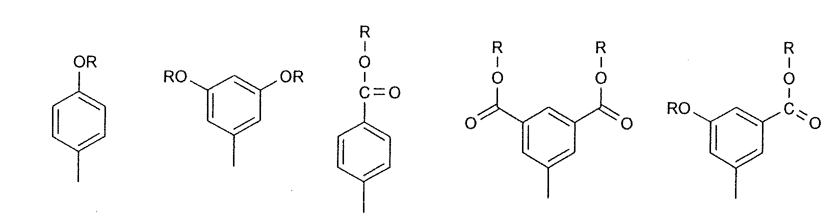

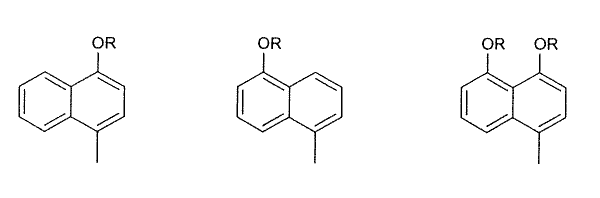

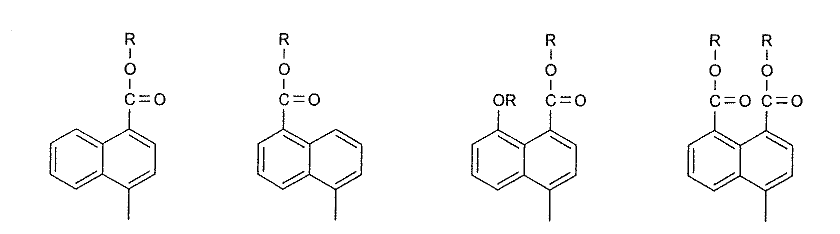

In the above formulae (15) to (17) and the above substituent Ar, any R, any RO- and any ROCO- as substituents can be suitably used so long as they are radiation-sensitive groups or groups having reactivity to the action of a chromophore active to radiation. Specific examples of R include hydrogen; tertiary hydrocarbon groups such as tert-butyl, adamantyl, etc.; substituents in which RO- constitutes a carbonic ester group such as tert-butoxycarbonyl; and substituents in which RO- constitutes an acetal group such as methoxymethyl, 1-ethoxyethyl, 1-phenoxyethyl, etc., and substituents shown below are also included as R. The substituents R may be used singly or in combination of at least two members of them so long as an advantage of the present invention is not impaired. 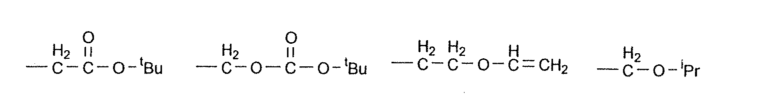 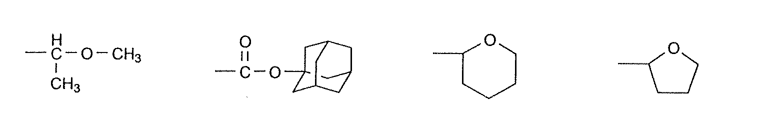 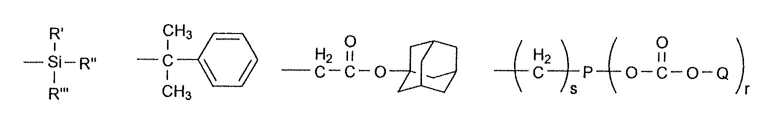 wherein each of R', R" and R'" is independently an aliphatic hydrocarbon group having 1 to 10 carbon atoms or an aromatic group, P is an aromatic group having a valence of (r + 1) and having 6 to 20 carbon atoms, Q is an organic group having 4 to 30 carbon atoms, r is an integer of 1 to 10 and s is an integer of 0 to 10.

In the organic group represented by 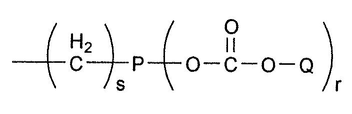 the numbers of carbon atoms of the aromatic group P and the organic group Q are preferably 6 to 10 and 4 to 20, respectively, and r and s are preferably 1 to 5 and 0 to 3, respectively.

Specifically, the following organic groups are included.

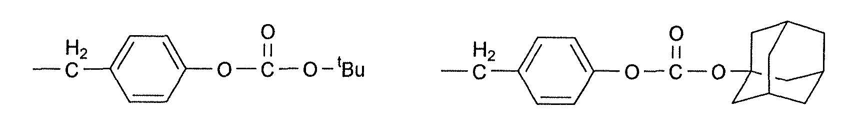

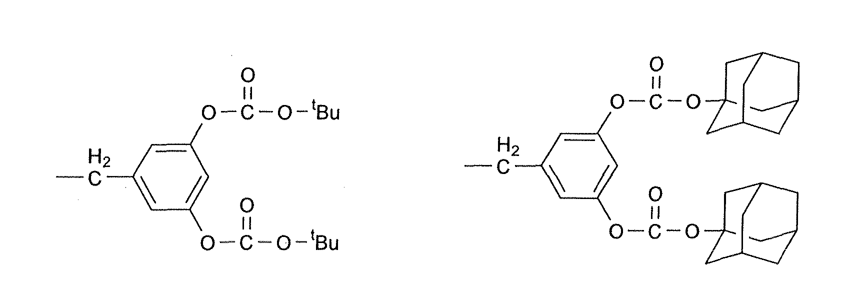

In the compound for use in the present invention, the content of basic impurities (e.g., ammonia, alkali metal ions of Li, Na, K, etc., and alkaline earth metal ions of Ca, Ba, etc.) is 10 ppm or less, preferably 2 ppm or less.

When the content of the basic impurities is decreased to 10 ppm or less, the photoresist base material comprising the above compound is remarkably improved in sensitivity to radiation, and as a result, a fine processing pattern by lithography using the photoresist composition can be fabricated.

The compound for use in the present invention is preferably in an amorphous state at room temperature, and the average diameter of the molecule thereof is smaller than the size of an intended pattern and is preferably 5 nm or less, more preferably 2 nm or less.

The average diameter is defined to be a diameter of the following true sphere. In a structure obtained by carrying out geometry optimization according to the AMI method of semiempirical orbit method program package MOPAC97, a diameter of a volume based on the van der Waals radius of a space occupied by the structure is assumed to be the true sphere.

The compound for use in the present invention can be synthesized by combining known reactions. When the compound contains impurities, the impurities can be removed, and the compound can be purified by a known way, as required.

In the present invention, the above compound is washed with an acidic aqueous solution and treated with an ion-exchange resin to be purified, whereby the content of the basic impurities can be decreased to 10 ppm or less. In this case, an optimum acidic aqueous solution and an optimum ion-exchange resin can be selected as required depending upon the amount and kind of the basic impurities to be removed or the kind of the compound to be treated. In the present invention, preferably, an acetic acid aqueous solution having a concentration of 0.01 to 10 mol/liter is used as an acidic aqueous solution, and a cation-exchange resin is used as an ion-exchange resin. For purification, particularly preferably, the washing is carried out using an acetic acid aqueous solution as an acidic aqueous solution, followed by treatment with a cation-exchange resin.

The thus-obtained compound is useful as a photoresist base material, particularly, as a photoresist base material for use in ultrafine processing with extreme ultra-violet or an electron beam.

That is, the compound for use in the present invention is in an amorphous state under conditions of use as a photoresist base material, generally, at room temperature as described above, so that the use thereof as a base material is preferred in view of application properties of a photoresist composition and strength of a photoresist film.

As described above, further, the compound for use in the present invention has a molecule whose average diameter is smaller than the value of line-edge roughness that is required in a desired pattern size, specifically, a size of 100 nm or less, particularly a size of 50 nm or less. When the above compound is used as a base material, therefore, the line-edge roughness can be suppressed to 2 nm or less, preferably 1 nm or less (3 σ) when it is used for 20 to 50 nm processing that is a characteristic feature of ultrafine processing based on extreme ultra-violet or an electron beam.

When such compounds are used as a photoresist base material, they may be used singly or in combination of two or more compounds of them so long as an advantage of the present invention is not impaired. Further, compounds formed by combining a plurality of such compounds with arbitrary substituent(s) may be used singly, or in combination of two or more thereof so long as an advantage of the present invention is not impaired.

The photoresist base material of the present invention can be used as one component for a photoresist composition.

The composition of the present invention is a liquid composition containing a solid content containing a photoresist base material comprising the above compound(s) and a solvent dissolving this solid content. In the present invention, the composition is required to be in a liquid state for uniformly applying a photoresist to a substrate, etc., on which ultrafine processing is to be applied.

The base material containing a radiation-sensitive group, provided by the present invention, contains a chromophore active to radiation, and it can exhibit its capability as a photoresist by itself, so that it is not especially necessary to add an additive to the solid content. However, when it is desirable to enhance the performance thereof as a photoresist or when a base material containing a group having reactivity to a chromophore active to radiation is used, there may be added an optically-acid-generating agent (PAG) or an optically-base-generating agent (PBG) as a chromophore.

As a PAG, known compounds represented by the following structures and other compounds having similar activities can be generally used. PAG can be selected among these as required depending upon the kind of the base material, the form and size of a desired fine pattern, and the like.

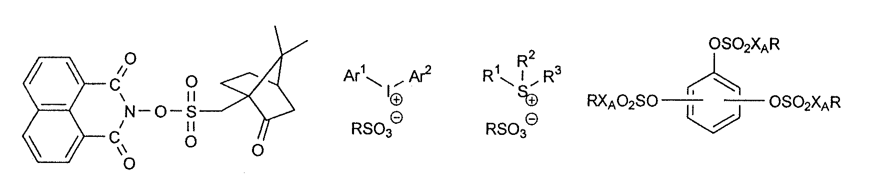

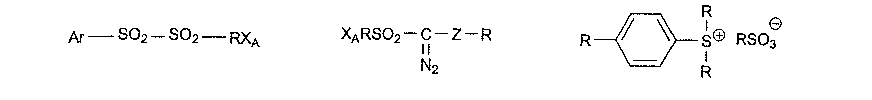

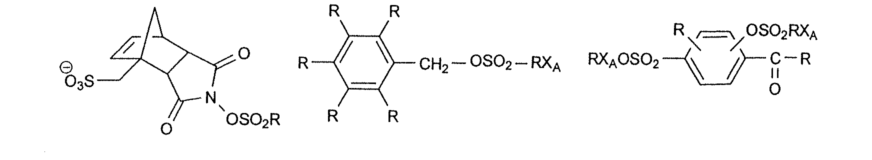

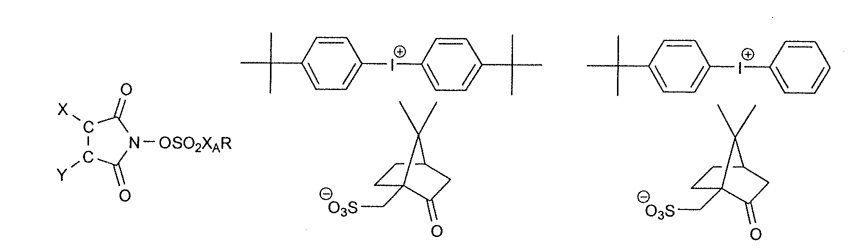

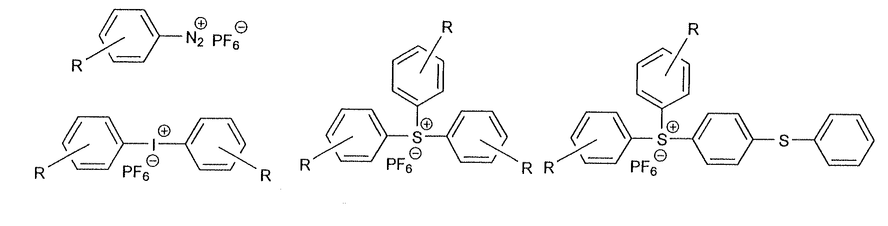

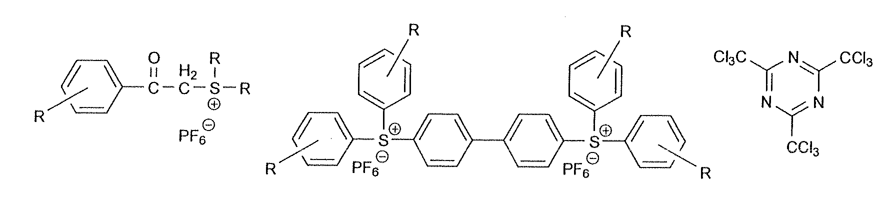

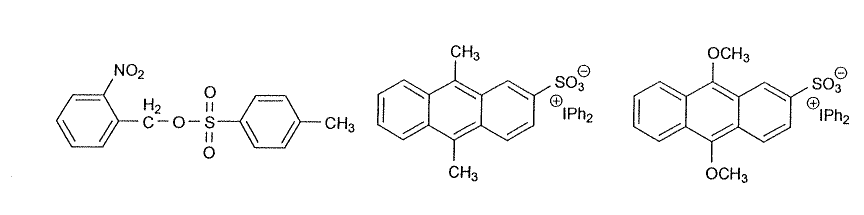

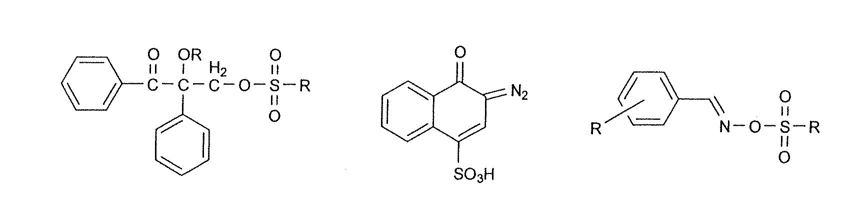

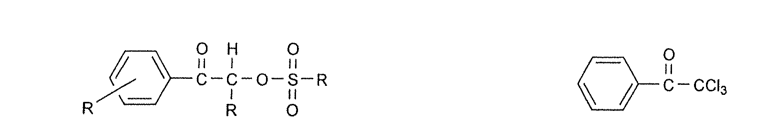

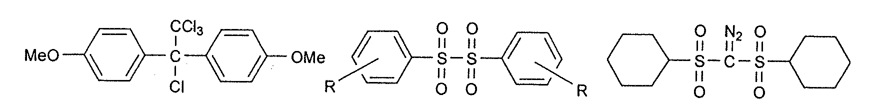

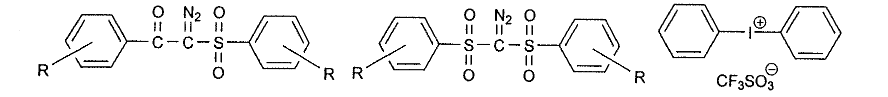

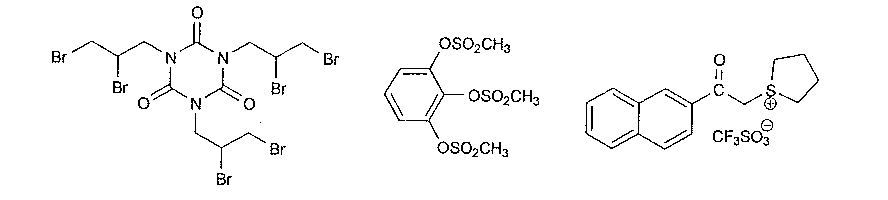

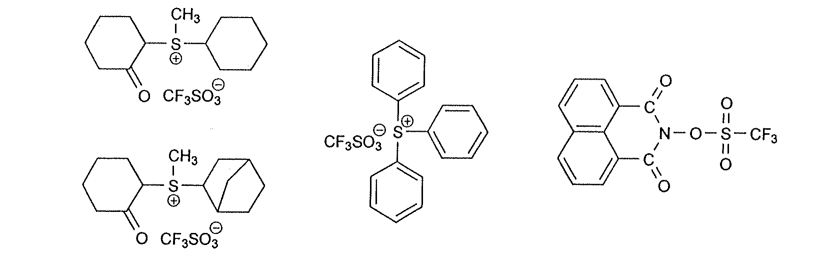

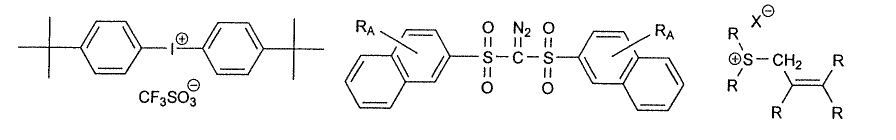

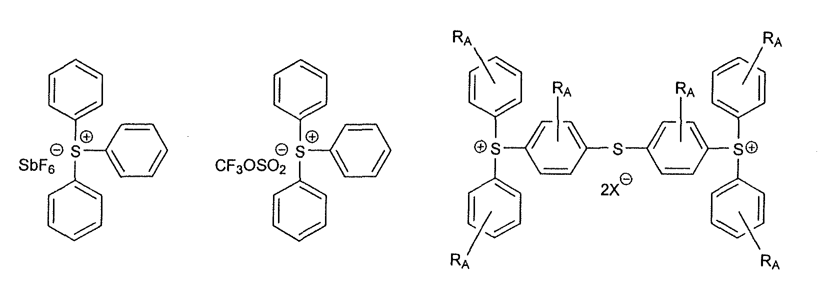

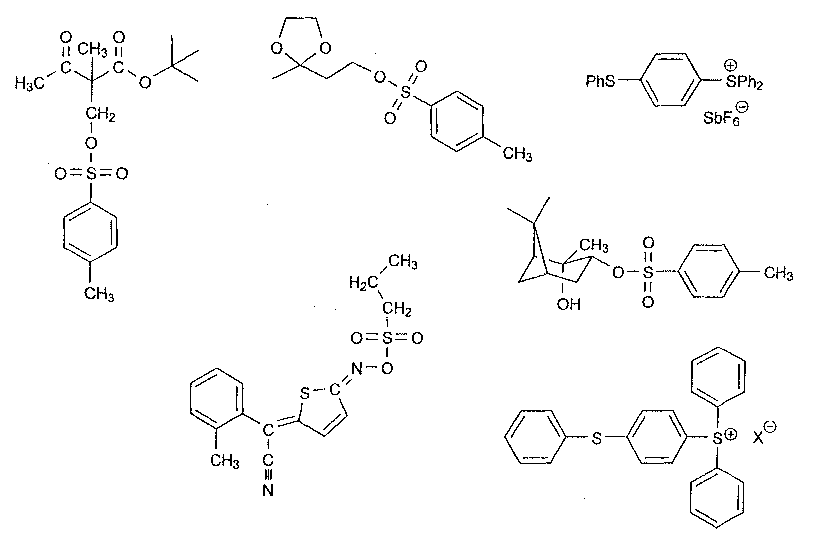

As a PBG, known compounds represented by the following structures and other compounds having similar activities can be generally used. PBG can be selected among these as required depending upon the kind of the base material, the form and size of a desired fine pattern, and the like. Further, when PBG is used as a chromophore, the amount thereof can be adjusted as required, while taking account of the proportion of basic impurities contained in the base material, to prevent excessive addition thereof which makes the reactivity beyond control.

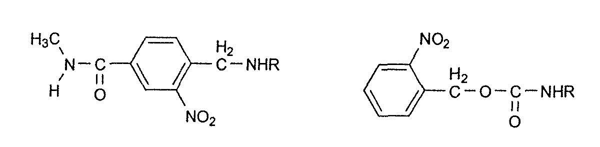

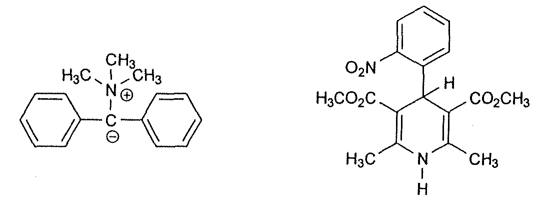

In the present invention, besides PAG and PBG, there can be added bases such as tetrabutylammonium hydroxide and salts thereof, an anti-striation agent, a plasticizer, a speed promoter, a photosensitizing agent, a sensitizing agent, an acid proliferation agent, an etching durability enhancer, and the like as required so long as an advantage of the present invention is not impaired.

Each component in the solid content may be a single component or a mixture of components having the same or different functions, or each may be a mixture of precursors of such components. The composition of the solid content and the amount ratio of components of the solid content can be adjusted as required depending upon the form, size, etc., of a desired fine pattern. Generally, an amount ratio, etc., similar to those of a conventional photoresist can be employed.

The solvent can be selected from those that are generally used as a solvent for a photoresist. Specifically, the solvent includes 2-methoxyethyl ether, glycols such as ethylene glycol monomethyl ether(2-methoxyethanol), propylene glycol monomethyl ether, 1-methoxy-2-propanol acetate, etc., lactic esters such as ethyl lactate, methyl lactate, etc., propionates such as methyl propionate, ethyl propionate, etc., cellosolve esters such as methyl cellosolve acetate, aromatic hydrocarbons such as toluene, xylene, etc., ketones such as methyl ethyl ketone, cyclohexanone, 2-heptanone, etc., and the like. The solvent can be selected among these solvents as required depending upon the solubility, film formability, etc., of the base material in the solvent.

The ratio of the solid content in the composition is generally adjusted to 1 to 40 % by weight based on the total weight of the composition. However, this ratio can be adjusted as required depending upon the kind of the base material.

The composition of the present invention is uniformly applied to a substrate such as silicon wafer, or any processible coating layer formed on a silicon wafer by a method such as spin coating, dip coating or painting. After the application, generally, the applied composition is dried until a photoresist coating layer comes to be a non-sticking layer, for example, up to 80 to 160°C, for removing the solvent. The heating condition can be adjusted as required depending upon the kind, etc., of the base material.

Then, the substrate whose photoresist coating layer has been non-sticking is irradiated with radiation with a photomask. After the exposure, the photoresist coating layer is baked to give or enhance a solubility difference between the exposed region and non-exposed region of the photoresist coating layer. The resultant substrate is developed with an alkali developer solution, or the like, for forming a relief image. By the above procedures, a processed ultrafine pattern is formed on the substrate.

When ultrafine processing with extreme ultra-violet or an electron beam is carried out with using the photoresist base material of the present invention and the composition thereof, a pattern with an isolated line having a size of 100 nm or finer, particularly, 50 nm or finer, a line/space (L/S) of 1/1, a hole, etc., can be formed with high sensitivity, a high contrast and a low line-edge roughness.

The photoresist base material and the composition thereof of the present invention are suitably used in the electric and the electronic field including semiconductor devices, the field of optics, and the like, whereby semiconductor devices such as ULSI can be remarkably improved in performances.

### Examples

The present invention will be specifically explained with reference to Examples hereinafter, while the present invention shall not be limited to the following Examples. Average diameters of base materials were determined according to the foregoing method.

### Example 1

### [Photoresist base material]

A three-necked flask (volume 500 ml) equipped with a dropping funnel, a Dimroth condenser tube and a thermometer, which had been fully dried and flushed with nitrogen gas, was hermetically charged with resorcinol (33 g, 300 mmol) and acetaldehyde (17 ml, 300 mmol) under nitrogen gas current, and under a slight pressure of nitrogen, distilled methanol (300 ml) was charged thereinto to prepare a methanol solution. This methanol solution was heated to 75°C in an oil bath with stirring. Then, 75 ml of concentrated hydrochloric acid was dropwise added gradually from the dropping funnel, followed by continuous stirring under heat at 75°C for 2 hours. After completion of the reaction, the reaction mixture was allowed to cool to room temperature, and then cooled in an ice bath. The reaction mixture was allowed to stand for 1 hour, to form an intended white crude crystal, and it was recovered by filtering. This crude crystal was washed with pure water (100 ml) twice, and recrystallized from a mixture of ethanol with water to purify it, and the thus-obtained crystal was dried under reduced pressure to give calix-[4]-resorcinarene having the above formula (15) in which all of R's were hydrogen atoms (16 g, yield 40.2 %). This product was used as a photoresist base material. The structure of this base material was determined on the basis of NMR measurement, IR measurement, elemental analysis, and the like. This base material had an average diameter of 0.88 nm and was in an amorphous state at room temperature.

### Example 2

### [Photoresist base material]

A catalytic amount of pyridinium p-toluene sulfonate was added to a toluene solution (40 ml) containing the calix-[4]-resorcinarene (2.07 g, 3.8 mmol) obtained in Example 1 and 1,2-dihydropyran (0.32 g, 3.8 mmol), and the mixture was stirred at 0°C for 10 minutes. Completion of the reaction was followed by extraction with diethyl ether, and an extract was washed with a sodium hydrogen carbonate aqueous solution, and the solvent was distilled off under reduced pressure to give a crude product in the form of a white precipitate. The crude product was recovered by filtering, washed with pure water and recrystallized for its purification, to give a calix-[4]-resorcinarene derivative having the above formula (15) in which all of R's were 1-tetrahydropyranyls (4.02 g, yield 87 %). This product was used as a photoresist base material. The structure of this base material was determined on the basis of NMR measurement, IR measurement, elemental analysis, and the like. This base material had an average diameter of 1.16 nm and was in an amorphous state at room temperature.

### Example 3

### [Photoresist base material]

Example 2 was repeated except that the amount of 1, 2-dihydropyran was changed to 0.16 g (1.9 mmol). As a result, there was obtained a calix-[4]-resorcinarene derivative having the above formula (15) in which 50 % of R's were 1-tetrahydropyranyls and 50 % of the R's were hydrogen atoms (2.74 g, yield 82 %). This product was used as a photoresist base material. The structure of this base material was determined on the basis of NMR measurement, IR measurement, elemental analysis, and the like. This base material had an average diameter of 1.04 nm and was in an amorphous state at room temperature.

### Example 4

### [Photoresist base material]

A two-necked flask (volume 100 ml) equipped with a Dimroth condenser tube and a thermometer, which had been fully dried and flushed with nitrogen gas, was hermetically charged with calix-[4]-resorcinarene (2.07 g, 3.8 mmol) obtained in Example 1, potassium carbonate (7.32 g, 30 mmol) and 18-crwon-6 (0.29 g, 1.08 mmol), and the atmosphere in the flask was replaced with nitrogen. Then, 38 ml of acetone was added to prepare a solution, then, tert-butyl bromoacetate (1.95 g, 10 mmol) was added, and in a nitrogen atmosphere, the mixture was refluxed under heat in an oil bath at 75°C for 24 hours with stirring. After completion of the reaction, the reaction solution was allowed to cool to room temperature, ice water was poured into the reaction solution, and the mixture was stirred for 1 hour to obtain a white precipitate. This precipitate was recovered by filtering and dried under reduced pressure to give a crude product of a calix-[4]-resorcinarene derivative having the above formula (15) in which 25 % of R's were tert-butyloxycarbonylmethyls and 75 % thereof were hydrogen atoms (2.23 g, yield 70 %). For removing potassium carbonate contained in a trace amount, then, it was dissolved in acetone (10 ml), the mixture was poured into an acetic acid aqueous solution (1 mol/liter, 300 ml) to give a white crystal. This crystal was recovered by filtering and dried under reduced pressure to give the above derivative purified (1.81 g, purification yield 81 %). This crystal was used as a base material. The structure of this base material was determined on the basis of TGA (weight of tert-butyloxycarbonylmethyl dissociated around 170°C), NMR measurement, IR measurement, elemental analysis, and the like. This base material had an average diameter of 0.99 nm and was in an amorphous state at room temperature.

### Example 5

### [Photoresist base material]

Sodium hydride having an amount of 3.5 equivalent weights (based on aliphatic hydroxyl groups) was gradually added to a tetrahydrofuran solution containing 1 equivalent weight of 1,3,5-trihydroxyadamantane, then, 3.5 equivalent weights (based on aliphatic hydroxyl groups) of 4-bromophenyl acetate was added, and the mixture was stirred for 5 hours while it was refluxed under heat. After the reaction mixture was cooled, diluted hydrochloric acid was gradually added to prepare an acidic aqueous solution, and the solution was again stirred for 1 hour while it was refluxed under heat. The thus-obtained precipitate was recovered by filtering, re-precipitated and washed with pure water for purification, to prepare an adamantane derivative having the above formula (3) in which all of Ar's were 4-hydroxyphenyls, and this product was used as a photoresist base material. This base material had an average diameter of 0.83 nm and was in an amorphous state at room temperature.

### Example 6

### [Photoresist base material]

To a tetrahydrofuran solution of 1,3-dihydroxyadamantane was gradually added a mixture of an equimolar amount of sodium hydride with an equimolar amount of 4-bromophenyl acetate, and the resulting mixture was stirred under reflux under heat for 5 hours. After the reaction mixture was cooled, pure water was added, the resultant precipitate was recovered by filtering and washed with pure water to give a mixture containing unreacted 1,3-dihydroxyadamantane, 1-(4'-acetoxyphenyloxy)-3-hydroxyadamantane and 1,3-di(4'-acetoxyphenyloxy)adamantine at a ratio of 1:2:1. The above mixture was purified to give 1-(4'-acetoxyphenyloxy)-3-hydroxyadamantane. To a tetrahydrofuran solution thereof was gradually added 1 equivalent weight (based on aliphatic hydroxyl groups) of sodium hydride, then, 1/3 equivalent weight (based on aliphatic hydroxyl groups) of 1,3,5-tribromobenzene was added, and the mixture was stirred under reflux under heat for 5 hours. After the reaction mixture was cooled, diluted hydrochloric acid was gradually added to prepare an acidic aqueous solution, and then the aqueous solution was again stirred under reflux under heat for 1 hour. The resultant precipitate was recovered by filtering and purified by re-precipitation and washing with pure water, to produce an adamantane having the above formula (11) in which all of Ar's were 4-hydroxyphenyls, and this product was used as a photoresist base material. This base material had an average diameter of 1.03 nm and was in an amorphous state at room temperature.

### Example 7

### [Photoresist base material]

Example 4 was repeated except that the amount of 18-crown-6 was changed to 0.58 g (2.16 mmol) and that the amount of tert-butyl bromoacetate was change to 3.9 g (20 mmol). As a result, there was obtained 3.52 g (yield 73 %), as a crude product, and 2.95 g (purification yield 83 %), as a purified product, of a calix-[4]-resorcinarene derivative having the above formula (15) in which 60 % of R's were tert-butyloxycarbonylmethyls and 40 % thereof were hydrogen atoms. This product was used as a base material. The structure of this base material was determined on the basis of TGA (weight of tert-butyloxycarbonylmethyl dissociated around 170°C), NMR measurement, IR measurement, elemental analysis, and the like. This base material had an average diameter of 1.11 nm and was in an amorphous state at room temperature.

### Example 8

### [Photoresist base material]

A suspension of 4-hydroxybenzyl alcohol (3.1 g, 25 mmol), di-tert-butyl-dicarbonate (5.45 g, 25 mmol) and potassium carbonate (3.45 g, 25 mmol) in acetone (100 ml) was stirred at room temperature under nitrogen atmosphere for 24 hours. After completion of the reaction, the solvent was distilled off under reduced pressure, followed by extraction with ether. An extract was washed with a 1M-NaOH aqueous solution, then, washed with a saturated sodium chloride aqueous solution, dried over anhydrous magnesium sulfate and filtered. The solvent was distilled off under reduced pressure, followed by purification by silica gel column chromatography (hexane/ethyl acetate), and 4-tert-butoxycarbonyloxybenzyl alcohol was thereby synthesized (1.52 g, yield 27 %). Then, a mixture solution of calix-[4]-resorcinarene (0.44 g, 0.80 mmol) obtained in Example 1 and 4-tert-butoxycarbonyloxybenzyl alcohol (1.5 g, 6.69 mmol) in a toluene/tetrahydrofuran (THF) (30 ml/20 ml) was stirred at 0°C under nitrogen atmosphere for 10 minutes. Then, 1,1'-azobis(N,N-dimethylformamide) (1.49 g, 8.7 mmol) and tri-n-butyl phosphine (1.76 g, 8.7 mmol) were added, and the mixture was stirred at 0°C under nitrogen atmosphere for 30 minutes. After completion of the reaction, an intended white crude crystal was formed and was recovered by filtering. This crude crystal was washed with pure water (100 ml) twice, then, re-crystallized from a mixture solution of ethanol with water for purification and dried under reduced pressure to give a calix-[4]-resorcinarene derivative having the above formula (15) in which all of R's were 4-(tert-butoxycarbonyloxy)benzyls (1.58 g, yield 90 %). This product was used as a base material. The structure of this base material was determined on the basis of NMR measurement, IR measurement, elemental analysis, and the like. This base material had an average diameter of 1.40 nm and was in an amorphous state at room temperature.

### Example 9

### [Photoresist base material]

Example 8 was repeated except that the amount of 4-tert-butoxycarbonyloxybenzyl alcohol was changed to 0.50 g (2.23 mmol), the amount of 1,1'-azobis(N,N-dimethylformamide) was changed to 0.50 g (2.9 mmol) and that the amount of tri-n-butyl phosphine was changed to 0.59 g (2.9 mmol). As a result, there was obtained a calix-[4]-resorcinarene derivative having the above formula (15) in which 25 % of R's were 4-(tert-butoxycarbonyloxy)benzyls and 75 % thereof were hydrogen atoms (0.65 g, yield 85 %). This product was used as a base material. The structure of this base material was determined on the basis of NMR measurement, IR measurement, elemental analysis, and the like. This base material had an average diameter of 1.06 nm and was in an amorphous state at room temperature.

### Comparative Example 1

### [Photoresist base material]

A commercially available polyhydroxystyrene (supplied by Aldrich, weight average molecular weight 10,000, molecular weight distribution 1.1) was used as a photoresist base material. While this base material was in an amorphous state at room temperature, it had an average diameter of 2.33 nm.

### Example 10

### [Photoresist composition]

A solid content containing 100 parts by weight of the base material prepared in Example 1, 2 parts by weight of di(t-butylphenyl)iodonium o-trifluoromethyl sulfonate (PAG) and 0.2 part by weight of tetrabutylammonium hydroxide lactate (base) was dissolved in ethyl lactate (solvent) such that the amount ratio thereof as a solid content was 5 % by weight, to prepare a photoresist solution (photoresist composition). The photoresist solution was spin-coated on a silicon wafer, and the thus-formed coating was dried under heat under reduced pressure to form a coating film having a thickness of 110 nm. Then, the substrate having this coating film was irradiated with extreme ultra-violet (wavelength 13.5 nm) through a photomask for a patterning test with an extreme ultra-violet exposure apparatus. Then, the coating film was baked at 110°C and treated with a 0.25 N tetrabutylammonium hydroxide aqueous solution to develop an imaged resist layer. As a result, there were not found any pattern collapse, etc., in a line/space having a 1/2 pitch width of 50 nm, and there was obtained an excellent pattern of which the line-edge roughness was substantially zero.

### Examples 11 - 18

### [Photoresist composition]

Example 10 was repeated except that the base material was replaced with calix-[4]-resorcinarene derivatives prepared in Examples 2 to 4 and 7 to 9 (Examples 11 to 16) or adamantane derivatives prepared in Examples 5 and 6 (Examples 17 and 18). As a result, there were not found any pattern collapse, etc., in a line/space having a 1/2 pitch width of 50 nm in any Example, and there were obtained excellent patterns of which the line-edge roughness was substantially zero.

### Comparative Example 2

### [Photoresist composition]

Example 10 was repeated except that the base material was replaced with polyhydroxystyrene of Comparative Example 1. As a result, there were not found any pattern collapse, etc., in a line/space having a 1/2 pitch width of 50 nm. However, line-edge roughness measurement showed an average of 4 nm, and there was not obtained a pattern that could be said to be excellent.

### Example 19

### [Photoresist composition]

A solid content containing 100 parts by weight of the base material prepared in Example 1, 20 parts by weight of (5-propylsulfonyloxyimino-5H-thiophen-2-ylidene)-2-methylphenylacetonitrile (PAG) and 0.2 part by weight of tetrabutylammonium hydroxide lactate (base) was dissolved in ethyl lactate (solvent) such that the amount ratio thereof as a solid content was 5 % by weight, to prepare a photoresist solution (photoresist composition). The photoresist solution was spin-coated on a silicon wafer, and the thus-formed coating was dried under heat under reduced pressure to form a coating film having a thickness of 110 nm. Then, the substrate having this coating film was irradiated with extreme ultra-violet (wavelength 13.5 nm) through a photomask for a patterning test with an extreme ultra-violet exposure apparatus. Then, the coating film was baked at 110°C and treated with a 0.25 N tetrabutylammonium hydroxide aqueous solution to develop an imaged resist layer. As a result, there were not found any pattern collapse, etc., in a line/space having a 1/2 pitch width of 50 nm, and there was obtained an excellent pattern of which the line-edge roughness was substantially zero.

### Examples 20 - 25

### [Photoresist composition]

Example 19 was repeated except that the base material was replaced with calix-[4]-resorcinarene derivatives prepared in Examples 2 to 4 and 7 to 9. As a result, there were not found any pattern collapse, etc., in a line/space having a 1/2 pitch width of 50 nm in any Example, and there were obtained excellent patterns of which the line-edge roughness was substantially zero.

### Example 26

### [Photoresist composition]

Example 19 was repeated except that a pattern was drawn directly on a substrate having a coating film with an electron beam irradiation apparatus (CABL9000 (trade name), supplied by CRESTEC CORPORATION) without irradiation through any photomask. As a result, there were not found any pattern collapse, etc., in a line/space having a 1/2 pitch width of 50 nm, and there was obtained an excellent pattern of which the line-edge roughness was substantially zero.

### Examples 27 - 32

### [Photoresist composition]

Example 26 was repeated except that the base material was replaced with calix-[4]-resorcinarene derivatives prepared in Examples 2 to 4 and 7 to 9. As a result, there were not found any pattern collapse, etc., in a line/space having a 1/2 pitch width of 50 nm, and there was obtained an excellent pattern of which the line-edge roughness was substantially zero.

### Example 33

### [Photoresist base material]

Di-tert-butyl carbonate (5.45 g, 25 mmol) was added to a suspension of 4-hydroxybenzyl alcohol (3.1 g, 25 mmol) and potassium carbonate (3.45 g, 25 mmol) in acetone (100 ml) at room temperature, and the mixture was stirred for 24 hours. After completion of the reaction, the acetone was distilled off under reduced pressure, and extraction with ether was carried out. This ether extract was washed with a 1 M sodium hydroxide aqueous solution and a saturated sodium chloride aqueous solution, then, anhydrous magnesium sulfate was added, and water was removed by filtering. The residue was concentrated under reduced pressure and purified by silica gel column chromatography, whereby 4-(tert-butoxycarbonyloxy)benzyl alcohol (1.52 g, yield 27 %) was obtained. Then, in an ice bath and under nitrogen current, the total amount (1.52 g, 6.7 mmol) of the thus-obtained 4-(tert-butoxycarbonyloxy)benzyl alcohol and 1,1,1-tris(4-hydroxyphenyl)ethane (0.61 g, 2 mmol) were hermetically charged, then, toluene (30 ml) and THF (20 ml) were poured thereinto, and the mixture was stirred for 10 minutes to prepare a mixture solution. Into this mixture solution were poured N,N,N',N'-tetramethylazodicarboxamide (1.5 g, 8.7 mmol) and tri-n-butyl phosphine (1.8 g, 8.7 mmol), and the mixture was stirred in a nitrogen atmosphere at 0°C for 30 minutes. After the reaction mixture was cooled, pure water was added, extraction with diethyl ether was carried out, and diethyl ether was distilled off under reduced pressure, to give a precipitate. This precipitate was purified by re-precipitation and washing with pure water, to give 1,1,1-tris[4-(4'-(tert-butoxycarbonyloxy)benzyloxy)phenyl]ethane (1.6 g, yield 87 %). This product was used as a photoresist base material. The structure of this base material was determined on the basis of NMR measurement, IR measurement, elemental analysis, and the like. This base material had an average diameter of 1.05 nm and was in an amorphous state at room temperature.

### Example 34

### [Photoresist base material]

Example 33 was repeated except that the 4-hydroxybenzyl alcohol was replaced with 3,5-dihydroxybenzyl alcohol (3.5 g, 25 mmol). As a result, there was obtained 1,1,1-tris[4-(3',5'-di(tert-butoxycarbonyloxy)benzyloxy)phenyl]ethane (2.3 g, yield 90 %). This product was used as a photoresist base material. The structure of this base material was determined on the basis of NMR measurement, IR measurement, elemental analysis, and the like. This base material had an average diameter of 1.17 nm and was in an amorphous state at room temperature.

### Example 35

### [Photoresist composition]

A solid content containing 100 parts by weight of the triaryl ethane compound synthesized in Example 33 as a base material, 2 parts by weight of di(t-butylphenyl)iodonium o-trifluoromethyl sulfonate (PAG) and 0.2 part by weight of tetrabutylammonium hydroxide lactate (base) was dissolved in ethyl lactate (solvent) such that the amount ratio thereof was 5 % by weight, to prepare a photoresist solution (photoresist composition). The photoresist solution was spin-coated on a silicon wafer, and the thus-formed coating was dried under heat under reduced pressure to form a coating film having a thickness of 110 nm. Then, the substrate having this coating film was irradiated with extreme ultra-violet (wavelength 13.5 nm) through a photomask for a patterning test with an extreme ultra-violet exposure apparatus. Then, the coating film was baked at 110°C and treated with a 0.25 N tetrabutylammonium hydroxide aqueous solution to develop an imaged resist layer. As a result, there were not found any pattern collapse, etc., when a line/space having a 1/2 pitch width of 50 nm was created, and there was obtained an excellent pattern of which the line-edge roughness was substantially zero.

### Example 36

### [Photoresist composition]

Example 35 was repeated except that the triarylethane compound synthesized in Example 33 and used in the photoresist solution was replaced with the triarylethane compound synthesized in Example 34 for use in a photoresist solution. As a result, there were not found any pattern collapse, etc., when a line/space having a 1/2 pitch width of 50 nm was created, and there was obtained an excellent pattern of which the line-edge roughness was substantially zero.

### Example 37

### [Photoresist composition]

A substrate having a similar coating film was prepared from the triarylethane compound synthesized in Example 33 in the same manner as in Example 35. With regard to this substrate having the coating film, a pattern was drawn directly thereon with the above electron beam irradiation apparatus without irradiation through any photomask. Then, the substrate having the coating layer was post-treated in the same manner as in Example 35, to develop the imaged resist layer. As a result, there were not found any pattern collapse, etc., when a line/space having a 1/2 pitch width of 50 nm was created, and there was obtained an excellent pattern of which the line-edge roughness was substantially zero.

### Example 38

### [Photoresist composition]

Example 37 was repeated except that the triarylethane compound synthesized in Example 33 and used in the photoresist solution was replaced with the triarylethane compound synthesized in Example 34 for use in the photoresist solution. As a result, there were not found any pattern collapse, etc., when a line/space having a 1/2 pitch width of 50 nm was created, and there was obtained an excellent pattern of which the line-edge roughness was substantially zero.

### Comparative Example 3

### [Photoresist composition]

Example 19 was repeated except that, as a base material, a copolymer of 4-hydroxystyrene (65 mol%)/styrene (20 mol%)/t-butyl acrylate (15 mol%) was synthesized by a conventional method and used. As a result, there were not found any pattern collapse, etc., in a line/space having a 1/2 pitch width of 50 nm. However, line-edge roughness measurement showed an average of 4 nm, and there was not obtained a pattern that could be said to be excellent.

### Comparative Example 4

### [Photoresist composition]

Example 26 was repeated except that the base material was replaced with the same copolymer as that used in Comparative Example 3. As a result, there were not found any pattern collapse, etc., in a line/space having a 1/2 pitch width of 50 nm. However, line-edge roughness measurement showed an average of 6 nm, and there was not obtained a pattern that could be said to be excellent.

### Example 39

### [Photoresist base material]

A two-necked flask (volume 100 ml) equipped with a Dimroth condenser tube and a thermometer, which had been fully dried and flushed with nitrogen gas, was hermetically charged with calix-[4]-resorcinarene (2.07 g, 3.8 mmol) obtained in Example 1, potassium carbonate (7.32 g, 30 mmol) and 18-crwon-6 (0.29 g, 1.08 mmol), and the atmosphere in the flask was replaced with nitrogen. Then, 38 ml of acetone was added to prepare a solution, then, tert-butyl bromoacetate (3.51 g, 18 mmol) was added, and in a nitrogen atmosphere, the mixture was refluxed under heat in an oil bath at 75°C for 24 hours with stirring. After completion of the reaction, the reaction solution was allowed to cool to room temperature, ice water was poured into the reaction solution, and the mixture was stirred for 1 hour to obtain a white precipitate. This precipitate was recovered by filtering and dried under reduced pressure to give a crude product of a calix-[4]-resorcinarene derivative having the above formula (15) in which 50 % of R's were tert-butyloxycarbonylmethyls and 50 % thereof were hydrogen atoms (3.04 g, yield 80 %). For removing potassium carbonate contained in a trace amount, then, it was dissolved in acetone (10 ml), the mixture was poured into an acetic acid aqueous solution (1 mol/liter, 300 ml) to give a white crystal. This crystal was recovered by filtering and dried under reduced pressure to give the above derivative purified (2.58 g, purification yield 85 %). This crystal was used as a base material. The structure of this base material was determined on the basis of TGA (weight of tert-butyloxycarbonylmethyl dissociated around 170°C), NMR measurement, IR measurement, elemental analysis, and the like. This base material had an average diameter of 1.10 nm and was in an amorphous state at room temperature. Further, this base material was quantitatively analyzed for a content of potassium ion contained therein with an inductively coupled plasma mass spectrometer, to show 1,000 to 1,500 ppm.

### Example 40

### [Purification of photoresist base material]

The calix-[4]-resorcinarene derivative (2 g) obtained in Example 39 was poured into an acetic acid aqueous solution (100 ml) adjusted to 1 mol/L, and the mixture was stirred in a suspension state at room temperature for 3 hours. This product was recovered by filtering and washed with pure water (100 mL) three times. The washed solid was dried at 80°C under reduced pressure to give a derivative treated with the acetic acid aqueous solution.

Then, a cation exchange resin (Amberlyst 15J-HG Dry, supplied by Hokkaido Organo Shoji Corporation??) substituted with acetone beforehand was packed in a glass column, the acetone solution of the treated derivative was caused to pass through it to concentrate the acetone solution under reduced pressure, the concentrated acetone solution was poured into ultra-pure water to carry out re-precipitation, and the thus-obtained precipitate was dried at 80°C under reduced pressure to give 1.5 g (yield 75 %) of an ion-exchange-treated derivative. This derivative was used as a photoresist base material. This base material was quantitatively analyzed for a content of potassium ion therein with an inductively coupled plasma mass spectrometer to show 0.5 to 1.5 ppm.

### Example 41

### [Photoresist composition]

A solid content containing 100 parts by weight of the calix-[4]-resorcinarene derivative prepared in Example 39 as a base material and 20 parts by weight of (5-propylsulfonyloxyimino-5H-thiophen-2-ylidene)-2-methylphenylacetonitrile (PAG) was dissolved in ethyl lactate (solvent) such that the amount ratio thereof was 20 % by weight, to prepare a photoresist solution (photoresist composition). The photoresist solution was spin-coated on a silicon wafer (1,000 rpm, 60 seconds), and the thus-formed coating was heated at 90°C for 180 seconds to form a coating film having a thickness of 1.2 µm. Then, the substrate having this coating film was irradiated with a UV ray at 100 mJ/cm² with a g-ray exposure apparatus (M-1S (trade name), supplied by Mikasa Co., Ltd.). Then, the coating film was baked at 100°C for 30 seconds and treated with a 0.25 N tetrabutylammonium hydroxide aqueous solution. A dissolving rate was calculated on the basis of a decrease in film thickness to show 28 nm/second.

### Example 42

### [Photoresist composition]

A solid content containing 100 parts by weight of the base material prepared in Example 40 and 10 parts by weight of (5-propylsulfonyloxyimino-5H-thiophen-2-ylidene)-2-methylphenylacetonitrile (PAG) was dissolved in 2-methoxyethanol (solvent) such that the amount ratio thereof was 20 % by weight, to prepare a photoresist solution (photoresist composition). The photoresist solution was spin-coated on a silicon wafer (1,000 rpm, 60 seconds), and the thus-formed coating was heated at 90°C for 180 seconds to form a coating film having a thickness of 1.2 µm. Then, the substrate having this coating film was irradiated with a UV ray at 80 mJ/cm² with the above g-ray exposure apparatus. Then, the coating film was baked at 100°C for 60 seconds and treated with a 0.25 N tetrabutylammonium hydroxide aqueous solution. A dissolving rate was calculated on the basis of a decrease in film thickness to show 1,350 nm/second.

### Example 43

### [Photoresist composition]

Example 42 was repeated except that the exposure dose by the g-ray exposure apparatus was changed to 5 mJ/cm². A dissolving rate was calculated on the basis of a decrease in film thickness to show 120 nm/second.

### Example 44

### [Photoresist composition]

A solid content containing 100 parts by weight of the base material prepared in Example 40 and 2 parts by weight of (5-propylsulfonyloxyimino-5H-thiophen-2-ylidene)-2-methylphenylacetonitrile (PAG) was dissolved in 2-methoxyethanol such that the amount ratio thereof was 20 % by weight, to prepare a photoresist solution (photoresist composition). The photoresist solution was spin-coated on a silicon wafer (1,000 rpm, 60 seconds), and the thus-formed coating was heated at 100°C for 180 seconds to form a coating film having a thickness of 1.2 µm. Then, the substrate having this coating film was irradiated with a UV ray with the above g-ray exposure apparatus. Then, the coating film was baked at 110°C for 60 seconds and treated with a 2.38 wt% tetrabutyl ammonium hydroxide aqueous solution for 60 seconds. While the intensity of the UV ray was changed, a solubility curve was prepared. When the sensitivity and contrast were estimated on the basis of the above curve, they were 19.2 mJ/cm² and 5.0, respectively. Further, when irradiation with the UV ray at 5 mJ/cm² was carried out with a photomask, there was obtained an image having a line width of 6 µm.

### Example 45

### [Photoresist composition]

There was synthesized a calix-[4]-resorcinarene derivative (average diameter: 1.06 nm, in an amorphous state at room temperature) having the above formula (15) in which 40 % of R's were tert-butyloxycarbonylmethyls and 60 % thereof were hydrogen atoms, in the same manner as in Example 39 except that the amount ratio of potassium carbonate and tert-butyl bromoacetate was changed to 4/5. The above derivative was purified in the same manner as in Example 40 and used as a base material. A photoresist solution was prepared using the above base material in the same manner as in Example 44, and a sensitivity curve thereof was prepared. As a result, when the sensitivity of a resist was estimated on the basis of the curve, it was 10.0 mJ/cm².

### Example 46

### [Photoresist composition]

There was synthesized a calix-[4]-resorcinarene derivative (average diameter: 1.00 nm, in an amorphous state at room temperature) having the above formula (15) in which 27 % of R's were tert-butyloxycarbonylmethyls and 73 % thereof were hydrogen atoms, in the same manner as in Example 39 except that the amount ratio of potassium carbonate and tert-butyl bromoacetate was changed to 3/5. The above derivative was purified in the same manner as in Example 40 and used as a base material. A photoresist solution was prepared using the above base material in the same manner as in Example 44, and a sensitivity curve thereof was prepared. As a result, when the sensitivity of a resist was estimated on the basis of the curve, it was 2.4 mJ/cm².

### Industrial Utility

According to the present invention, there can be provided a photoresist base material that enables ultrafine processing by extreme ultra-violet, etc., with high sensitivity, a high contrast and a low line-edge roughness, a method for purification thereof and a photoresist composition.

## Claims

**1.** A photoresist base material comprising an extreme ultra-violet reactive organic compound represented by the following general formula (1), 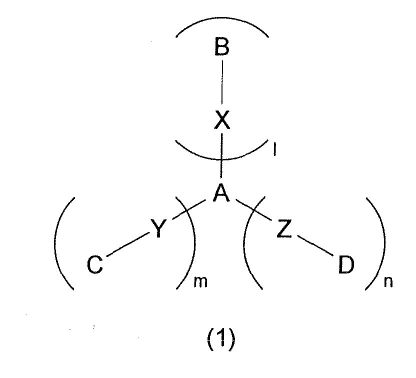 wherein A is a central structure that is an aliphatic group having 1 to 50 carbon atoms, an aromatic group having 6 to 50 carbon atoms, an organic group containing said aliphatic group and said aromatic group together or an organic group having a cyclic structure formed by repetition of these groups, each of B, C and D is independently an extreme ultra-violet reactive group, a group having reactivity to the action of chromophore active to extreme ultra-violet, or a C₁ to C₅₀ aliphatic group, C₆ to C₅₀ aromatic group, an organic group containing said aliphatic group and said aromatic group together or a substituent having a branched structure, containing such a reactive group, each of X, Y and Z is independently a single bond or an ether bond, each of l, m and n is independently an integer of 0 to 5 satisfying l + m + n ≥ 1, and A, B, C and D may contain a substituent having a heteroatom.

**2.** The photoresist base material as recited in claim 1, wherein said organic compound reactive to extreme ultra-violet is in an amorphous state at room temperature and has a molecule whose average diameter is 2 nm or less.

**3.** The photoresist base material as recited in claim 1 or 2, wherein A is an organic group represented by 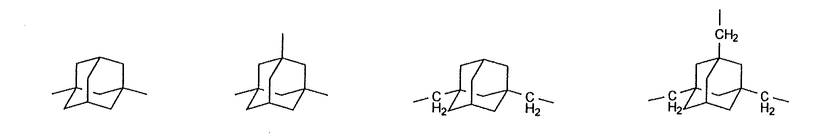 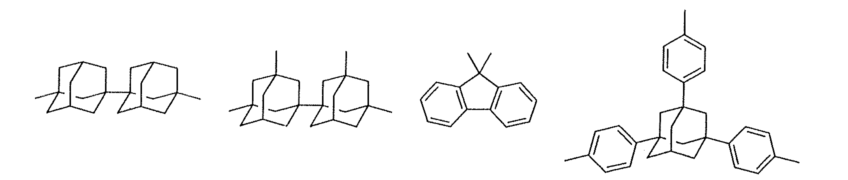 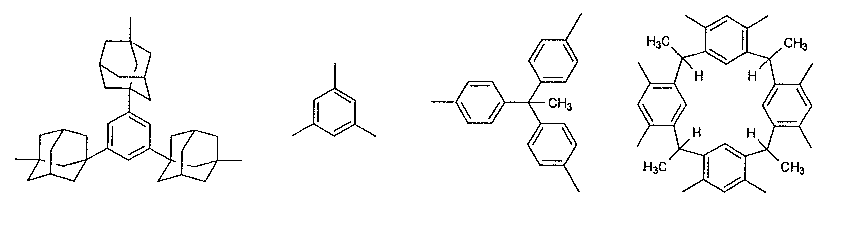 each of B, C and D is an extreme ultra-violet reactive group, a group having reactivity to the action of chromophore active to extreme ultra-violet, or an organic group represented by 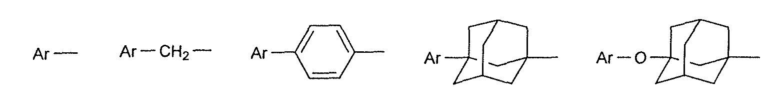 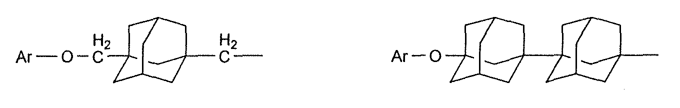 wherein Ar is a phenyl or naphthyl group substituted with RO- and/or ROCO- in which R, RO- and ROCO are extreme ultra-violet reactive groups or groups having reactivity to the action of a chromophore active to extreme ultra-violet, and
X, Y and Z are ether bonds.

**4.** The photoresist base material as recited in claim 3, wherein A is an organic group represented by 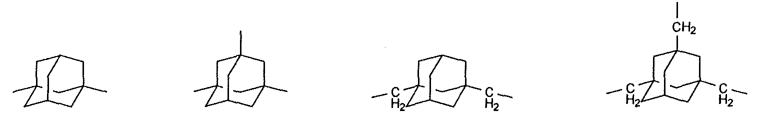 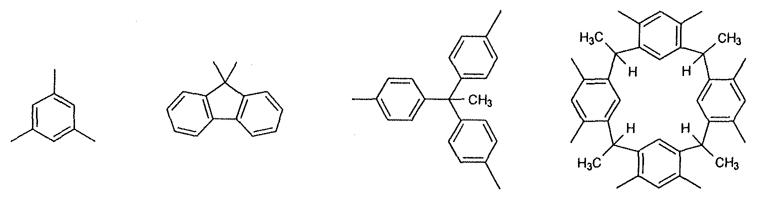 each of B, C and D is a hydrogen atom, tert-butyl, tert-butyloxycarbonylmethyl, tert-butyloxycarbonyl, 1-tetrahydropyranyl, 1-tetrahydrofuranyl, 1-ethoxyethyl, 1-phenoxyethyl, an organic group represented by 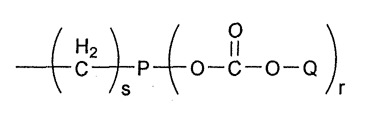 in which P is an aromatic group having a valence of (r + 1) and having 6 to 20 carbon atoms, Q is an organic group having 4 to 30 carbon atoms, r is an integer of 1 to 10 and s is an integer of 0 to 10, or an organic group represented by 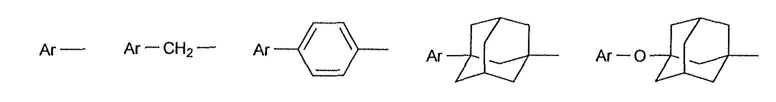 in which Ar is a phenyl or naphthyl group substituted with RO- and/or ROCO- in which R is hydrogen, tert-butyl, tert-butyloxycarbonylmethyl, tert-butyloxycarbonyl, 1-tetrahydropyranyl, 1-tetrahydrofuranyl, 1-ethoxyethyl, 1-phenoxyethyl, an organic group represented by 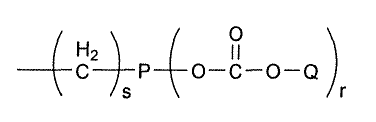 in which P is an aromatic group having a valence of (r + 1) and having 6 to 20 carbon atoms, Q is an organic group having 4 to 30 carbon atoms, r is an integer of 1 to 10 and s is an integer of 0 to 10,
and X, Y and Z are ether bonds.

**5.** The photoresist base material as recited in claim 4, wherein A is an organic group represented by 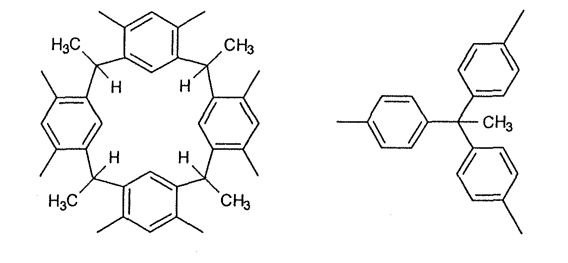 each of B, C and D is a hydrogen atom, tert-butyl, tert-butyloxycarbonylmethyl, tert-butyloxycarbonyl, 1-tetrahydropyranyl, 1-tetrahydrofuranyl, 1-ethoxyethyl, 1-phenoxyethyl or an organic group represented by 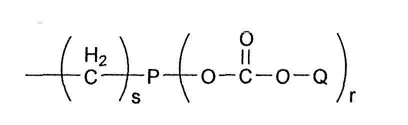 in which P is an aromatic group having a valence of (r + 1) and having 6 to 20 carbon atoms, Q is an organic group having 4 to 30 carbon atoms, r is an integer of 1 to 10 and s is an integer of 0 to 10,
and X, Y and Z are ether bonds.

**6.** A photoresist base material comprising a radiation-sensitive organic compound represented by the following general formula (1), 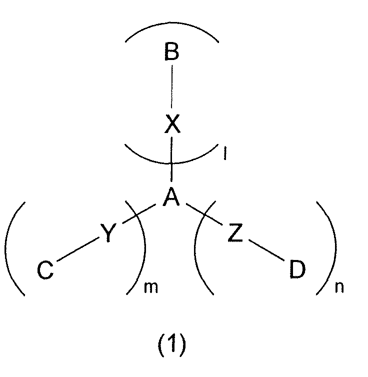 wherein A is an organic group represented by 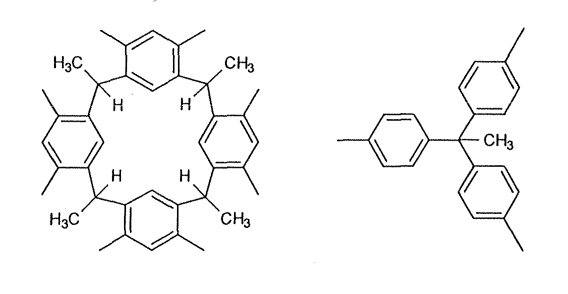 each of B, C and D is independently an organic group represented by 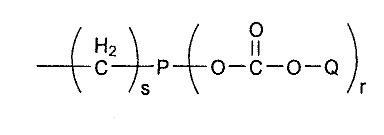 in which P is an aromatic group having a valence of (r + 1) and having 6 to 20 carbon atoms, Q is an organic group having 4 to 30 carbon atoms, r is an integer of 1 to 10 and s is an integer of 0 to 10, and
l + m + n = 3 or 8.

**7.** The photoresist base material as recited in claim 6, wherein the organic group represented by 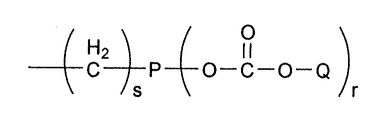 is 4-(tert-butoxycarbonyloxy)benzyl or 3,5-di(tert-butoxycarbonyloxy)benzyl.

**8.** The photoresist base material as recited in claim 6, wherein the radiation is extreme ultra-violet or electron beam.

**9.** The photoresist base material as recited in any one of claims 1 to 8, wherein at least one of B, C and D is a hydrogen atom and X, Y and Z are ether bonds.

**10.** The photoresist base material as recited in any one of 1 to 8, which has a basic impurity content of 10 ppm or less.

**11.** A photoresist composition containing a solid content containing the photoresist base material recited in any one of claims 1 to 8 and a solvent.

**12.** A photoresist composition comprising a solid content containing the photoresist base material recited in claim 10 and a solvent.

**13.** The photoresist composition as recited in claim 11 or 12, which further contains an optically-acid-generating agent.

**14.** A method for purification of a photoresist base material, which comprises washing the photoresist base material recited in any one of claims 1 to 8 with an acidic aqueous solution and treating the material with an ion-exchange resin.

**15.** The method for purification of a photoresist base material as recited in claim 14, wherein said acidic aqueous solution is an acetic acid aqueous solution.

**16.** A method for improvement of the photoresist base material recited in any one of claims 1 to 8 in radiation sensitivity, which comprises decreasing the content of basic impurities to 10 ppm or less.

**17.** A method for fine processing by lithography, which uses the photoresist composition recited in claim 11 or 12.

**18.** A semiconductor device fabricated using the photoresist composition recited in claim 11 or 12.

**19.** An organic compound represented by the following general formula (1), 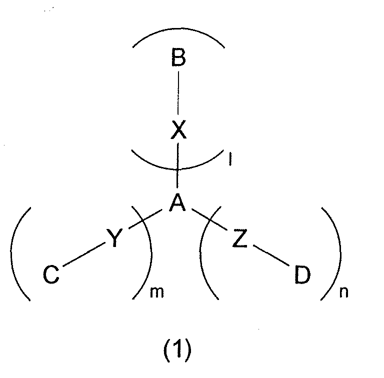 wherein A is an organic group represented by 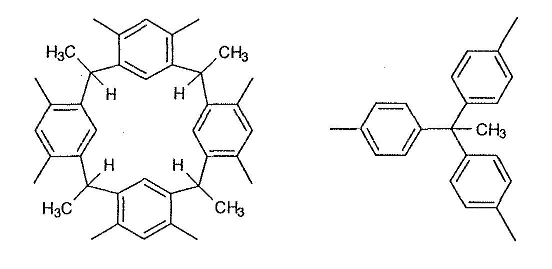 each of B, C and D is independently an organic group represented by 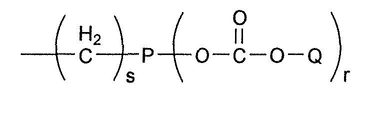 in which P is an aromatic group having a valence of (r + 1) and having 6 to 20 carbon atoms, Q is an organic group having 4 to 30 carbon atoms, r is an integer of 1 to 10 and s is an integer of 0 to 10,
and
l + m + n = 3 or 8.

**20.** The organic compound as recited in claim 19, which has a basic impurity content of 10 ppm or less.

**21.** A method for purification of an organic compound, which comprises washing the organic compound recited in claim 19 with an acidic aqueous solution and treating the compound with an ion-exchange resin.

## Amended claims

### Amended claims under Art. 19.1 PCT

1. (Amended) A photoresist base material comprising an extreme ultra-violet reactive organic compound represented by the following general formula (1), 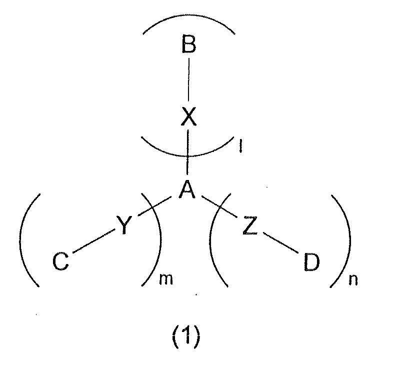 wherein A is an organic group represented by 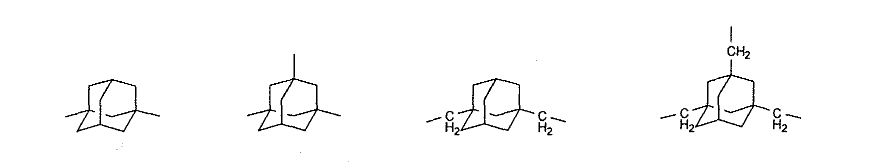 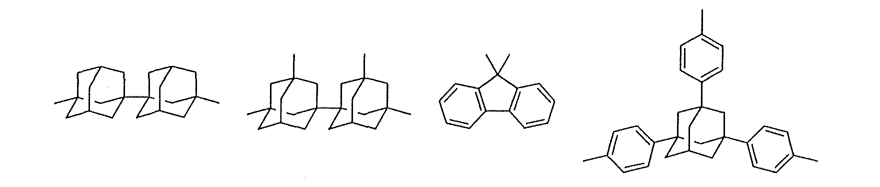 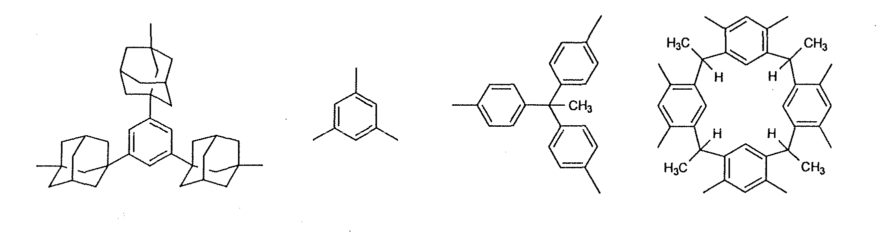 each of B, C and D is independently an extreme ultra-violet reactive group, a group having reactivity to the action of chromophore active to extreme ultra-violet or an organic group represented by 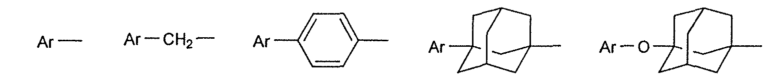 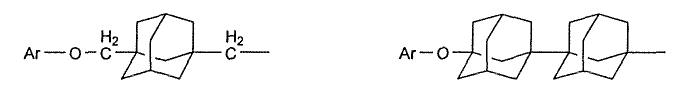 wherein Ar is a phenyl or naphthyl group substituted with RO- and/or ROCO- in which R, RO- and ROCO are extreme ultra-violet reactive groups or groups having reactivity to the action of a chromophore active to extreme ultra-violet,
each of X, Y and Z is independently a single bond or an ether bond, and
l + m + n = 2, 3, 4 or 8.

2. The photoresist base material as recited in claim 1, wherein said extreme ultra-violet reactive organic compound is in an amorphous state at room temperature and has a molecule whose average diameter is 2 nm or less.

3. (Amended) The photoresist base material as recited in claim 1, wherein A is an organic group represented by 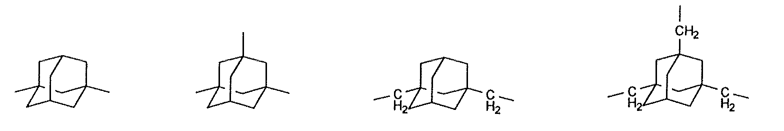 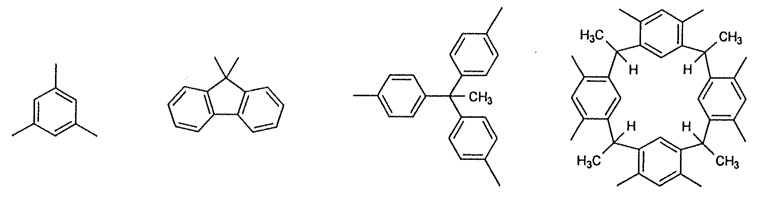 each of B, C and D is a hydrogen atom, tert-butyl, tert-butyloxycarbonylmethyl, tert-butyloxycarbonyl, 1-tetrahydropyranyl, 1-tetrahydrofuranyl, 1-ethoxyethyl, 1-phenoxyethyl, an organic group represented by 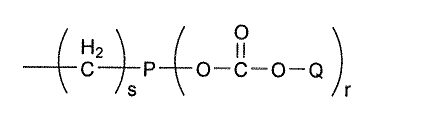 in which P is an aromatic group having a valence of (r + 1) and having 6 to 20 carbon atoms, Q is an organic group having 4 to 30 carbon atoms, r is an integer of 1 to 10 and s is an integer of 0 to 10, or an organic group represented by 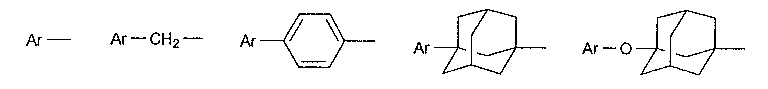 in which Ar is a phenyl or naphthyl group substituted with RO- and/or ROCO- in which R is hydrogen, tert-butyl, tert-butyloxycarbonylmethyl, tert-butyloxycarbonyl, 1-tetrahydropyranyl, 1-tetrahydrofuranyl, 1-ethoxyethyl, 1-phenoxyethyl or an organic group represented by 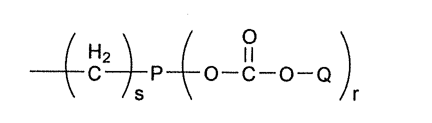 in which P is an aromatic group having a valence of (r + 1) and having 6 to 20 carbon atoms, Q is an organic group having 4 to 30 carbon atoms, r is an integer of 1 to 10 and s is an integer of 0 to 10,
and each of X, Y and Z is independently a single bond or an ether bond.

4. (Amended) The photoresist base material as recited in claim 3, wherein A is an organic group represented by 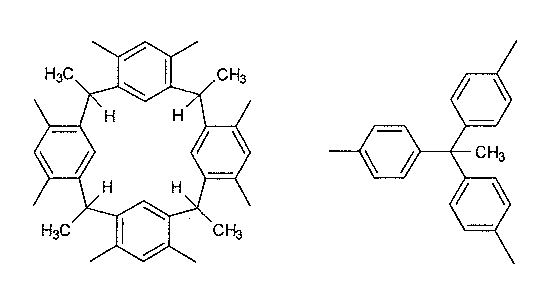 each of B, C and D is a hydrogen atom, tert-butyl, tert-butyloxycarbonylmethyl, tert-butyloxycarbonyl, 1-tetrahydropyranyl, 1-tetrahydrofuranyl, 1-ethoxyethyl, 1-phenoxyethyl or an organic group represented by 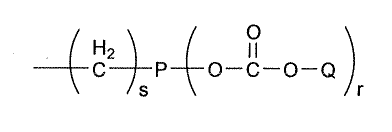 in which P is an aromatic group having a valence of (r + 1) and having 6 to 20 carbon atoms, Q is an organic group having 4 to 30 carbon atoms, r is an integer of 1 to 10 and s is an integer of 0 to 10,
and X, Y and Z are ether bonds.

5. (Amended) A photoresist base material comprising a radiation-sensitive organic compound represented by the following general formula (1), 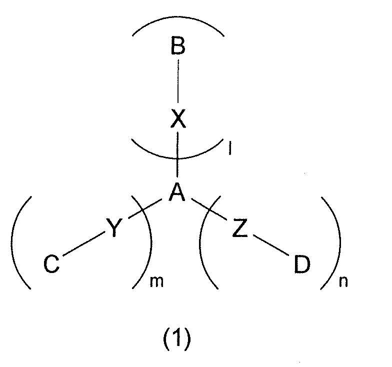 wherein A is an organic group represented by 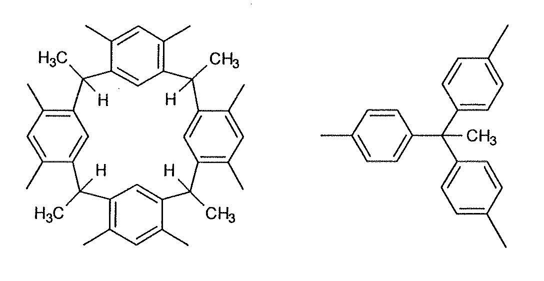 each of B, C and D is independently tert-butyloxycarbonylmethyl, tert-butyloxycarbonyl or an organic group represented by 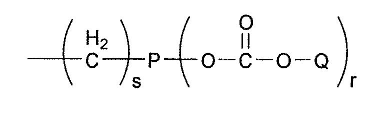 in which P is an aromatic group having a valence of (r + 1) and having 6 to 20 carbon atoms, Q is an organic group having 4 to 30 carbon atoms, r is an integer of 1 to 10 and s is an integer of 0 to 10,
each of X, Y and Z is independently a single bond or an ether bond, and
l + m + n = 3 or 8.

6. (Amended) The photoresist base material as recited in claim 5, wherein the organic group represented by 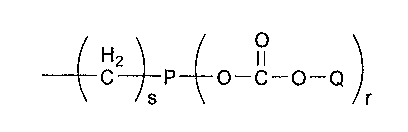 is 4-(tert-butoxycarbonyloxy)benzyl or 3,5-di(tert-butoxycarbonyloxy)benzyl.

7. (Amended) The photoresist base material as recited in claim 5, wherein the radiation is extreme ultra-violet or electron beam.

8. (Amended) The photoresist base material as recited in any one of claims 1 to 7, wherein at least one of B, C and D is a hydrogen atom and X, Y and Z are ether bonds.

9. (Amended) The photoresist base material as recited in any one of 1 to 7, which has a basic impurity content of 10 ppm or less.

10. (Amended) A photoresist composition comprising a solid content containing the photoresist base material recited in any one of claims 1 to 7 and a solvent.

11. (Amended) A photoresist composition comprising a solid content containing the photoresist base material recited in claim 9 and a solvent.

12. (Amended) The photoresist composition as recited in claim 10 or 11, which further comprises an optically-acid-generating agent.

13. (Amended) A method for purification of a photoresist base material, which comprises washing the photoresist base material recited in any one of claims 1 to 7 with an acidic aqueous solution and treating the material with an ion-exchange resin.

14. (Amended) The method for purification of a photoresist base material as recited in claim 13, wherein said acidic aqueous solution is an acetic acid aqueous solution.

15. (Amended) A method for improvement of the photoresist base material recited in any one of claims 1 to 7 in radiation sensitivity, which comprises decreasing the content of basic impurities to 10 ppm or less.

16. (Amended) A method for fine processing by lithography, which uses the photoresist composition recited in claim 10 or 11.

17. (Amended) A semiconductor device fabricated using the photoresist composition recited in claim 10 or 11.

18. (Amended) An organic compound represented by the following general formula (1), 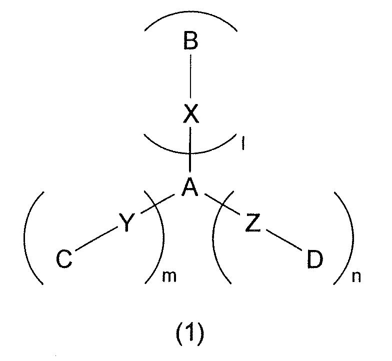 wherein A is an organic group represented by 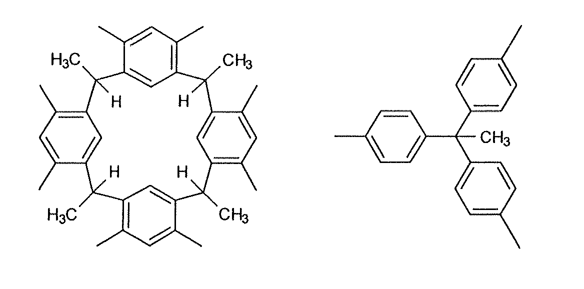 each of B, C and D is independently tert-butyloxycarbonylmethyl, tert-butyloxycarbonyl or an organic group represented by 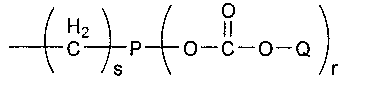 in which P is an aromatic group having a valence of (r + 1) and having 6 to 20 carbon atoms, Q is an organic group having 4 to 30 carbon atoms, r is an integer of 1 to 10 and s is an integer of 0 to 10,
each of X, Y and Z is independently a single bond or an ether bond, and
l + m + n = 3 or 8.

19. (Amended) The organic compound as recited in claim 18, which has a basic impurity content of 10 ppm or less.

20. (Amended) A method for purification of an organic compound, which comprises washing the organic compound recited in claim 18 with an acidic aqueous solution and treating the compound with an ion-exchange resin.
